# EUROPEAN PATENT SPECIFICATION

(11) **EP 0 625 903 B1**
(45) Date of publication and mention of the grant of the patent: **12.08.1998**
(21) Application number: 93906084.4
(22) Date of filing: 19.02.1993
(51) Int. Cl.: A61K 31/54, A61K 31/38, A61K 31/425

(54) **TOPICAL ANTIGLAUCOMA COMPOSITIONS COMPRISING CARBONIC ANHYDRASE INHIBITORS AND BETA-BLOCKERS**
Topische Antiglaukoma Zusammensetzungen, enthaltend Karbonhydrasehemmer und Beta-Blocker
COMPOSITIONS ANTIGLAUCOME A USAGE LOCAL COMPRENANT DES INHIBITEURS D'ANHYDRASE CARBONIQUE ET DE BETA-BLOQUANTS

(30) Priority: 21.02.1992 US 839869
(43) Date of publication of application: 30.11.1994
(73) Proprietor: ALCON LABORATORIES, INC., Fort Worth Texas 76134-2099 (US)
(72) Inventor: DEAN, Thomas Robert, Weatherford, TX 76087 (US); DESANTIS, Louis, Jr., Fort Worth, TX 76109 (US)
(74) Representative: Keller, Günter, Dr.
(86) International application number: US9301487
(87) International publication number: WO9316701

(56) References cited:
- EP-A- 0 429 732
- EP-A- 0 452 151
- EP-A- 0 495 421
- EP-A- 0 507 224
- EP-A- 0 509 752
- WO-A-91/15486
- US-A- 3 655 663
- US-A- 4 731 368
- US-A- 4 863 922
- INTERNATIONAL OPHTHALMOLOGY vol. 15, no. SUP. , 1991 page 11-12 F.P. GUNNING 'Medical treatment of glaucoma: developments in research on topical carbonic anhydrase inhibitors.'
- FORTSCHR. OPHTHALMOL. vol. 88, no. 6 , 1991 pages 846 - 847 M. PFEIFFER 'Additive Wirkung von Timolol und dem lokalen Karboanhydrasehemmer MK-417 (Sezolamid).'
- J. MED. CHEM. vol. 15, no. 6 , 1972 pages 651 - 655 B.K. WASSON 'beta-Adrenergic blocking agents. 3-(3-Substituted-amino-2-hydroxypr opoxy)-4-substituted-1,2,5-thiadiazoles.'

## Description

This application is a continuation-in-part of U.S. Patent Application Serial No. 07/839,869, filed February 21, 1992.

### Background of the Invention

The present invention relates to the field of ophthalmology. In particular, the invention relates to the treatment of glaucoma and associated elevations of intraocular pressure and to the treatment of ocular hypertension associated with other diseases or conditions.

Although the underlying causes of glaucoma are not understood, its symptoms often include elevated intraocular pressure, which may be caused either by over-production or inadequate outflow of aqueous humor. If left untreated, or if inadequately treated, glaucoma can lead to blindness or significant loss of vision. There is therefore a continuing need for therapies which control the elevated intraocular pressure associated with glaucoma.

There are currently a number of drugs utilized in the treatment of glaucoma, including: miotics (e.g., pilocarpine, carbachol and acetylcholinesterase inhibitors); sympathomimetics (e.g., epinephrine, dipivalylepinephrine and para-amino clonidine); beta-blockers (e.g., betaxolol, levobunolol and timolol); and carbonic anhydrase inhibitors (e.g., acetazolamide, methazolamide and ethoxzolamide). Miotics and sympathomimetics are believed to lower intraocular pressure ("IOP") by increasing the outflow of aqueous humor, while beta-blockers and carbonic anhydrase inhibitors are believed to lower IOP by decreasing the formation of aqueous humor. All four types of drugs have potentially serious side effects. Miotics such as pilocarpine can cause blurring of vision and other visual side effects, which may lead either to decreased patient compliance or to termination of therapy. Carbonic anhydrase inhibitors can also cause serious side effects which affect patient compliance and/or necessitate the withdrawal of treatment. Moreover, at least one beta-blocker, timolol, has increasingly become associated with serious pulmonary side effects attributable to its effect on beta-2 receptors in pulmonary tissue.

A significant number of glaucoma patients require the administration of more than one type of drug in order to achieve therapeutic control over their IOP. That is, a single drug does not provide adequate control of IOP in these patients. Treatment which includes the use of two or more of the above-cited classes of drugs requires the patient to apply the compositions to the affected eye(s) in separate, spaced dosages several times a day. Patient compliance with such complicated dosage regimens can be very poor, particularly with elderly patients. Since the majority of glaucoma patients are elderly, this is a significant problem.

In light of the foregoing circumstances, it is clear that a need exists for new, more potent anti-glaucoma compositions which avoid or reduce the above-cited side effects, while increasing patient compliance. The present invention is directed to such compositions.

### Summary of the Invention

As mentioned above, two or more different types of drugs are sometimes required to achieve therapeutic control of intraocular pressure. The use of a combination of drugs from two of the above-mentioned four classes of drugs has the advantage of reducing intraocular pressure via two different mechanisms. In particular, although both beta-blockers and carbonic anhydrase inhibitors are believed to lower IOP by decreasing the formation of aqueous humor, each of these classes of drugs operates by different mechanisms; therefore, a combination of at least one beta-blocker and at least one carbonic anhydrase inhibitor ("CAI"), when formulated in a composition also including anionic mucomimetic polymers and finely-divided drug carrier substrates ("DCS" - defined below) provides reduction of IOP and additionally provides comfortable, sustained-released compositions.

It has also been found, quite unexpectedly, that certain CAI's which have exceptionally low inherent aqueous solubility are effective in lowering and controlling IOP when dosed topically to the eye as suspensions, preferably having neutral pH. These formulations have been found to be very well tolerated, and appear to be significantly more comfortable and have fewer side effects than solutions of CAI's which have higher inherent aqueous solubility (these solutions are typically formulated at a pH between about 5.0 and 6.0). As such, combinations of beta-blockers with these low aqueous solubility CAI's formulated as suspensions will provide comfortable and effective medicaments for lowering and controlling IOP. The additional inclusion of anionic mucomimetic polymers and/or DCS will provide sustained release formulations.

Thus, the present invention is directed to such anti-glaucoma compositions, as well as methods of controlling IOP utilizing these compositions.

### Detailed Description of the Invention

The anti-glaucoma compositions of the present invention comprise a combination of one or more beta-blockers and one or more carbonic anhydrase inhibitors, formulated as suspensions having a pH between about 5.0 and about 7.8, preferably formulated as suspensions having a pH between about 6.8 and about 7.8. The anti-glaucoma compositions of the present invention may additionally contain anionic mucomimetic polymers and/or DCS to provide sustained release.

The beta-blockers which are useful in the compositions of the present invention include all beta-blockers which demonstrate the requisite cation charge and intraocular pressure effect. Such beta-blockers are typically represented by the following generic structure:

R'₁-O-CH₂-CH(OH)-CH₂-NR'₂R'₃ (I)

wherein:
- R'₁: is a substituted or unsubstituted cyclic or aliphatic moiety; cyclic moieties include mono- and polycyclic structures which may contain one or more heteroatoms selected from C, N, and O; and
- R'₂ and R'₃: are independently selected from H and substituted and unsubstituted alkyl.

With regard to Structure (I), above, the following references are hereby incorporated by reference herein: Annual Reports in Medicinal Chemistry, 14:81-87 (1979); J. Med. Chem., 26:1570-1576 (1983); ibid., 27:503-509 (1984); ibid., 26:7-11 (1983); ibid., 26:1561-1569 (1983); ibid., 26:1109-1112 (1983); ibid., 26:950-957 (1983); ibid., 26:649-657; and ibid., 26:352-357 (1983). Representative beta-blockers include the racemic and enantiomeric forms of: betaxolol, timolol, metoprolol, befunolol, falintolol, levobunolol, carteolol, mepindolol, pindolol, bisoprolol, bopindolol, atenolol, arotinolol, acebutolol, nadolol, celiprolol, metipranolol, bevantolol, ICI 118,551, pamatolol, penbutolol, toliprolol, tiprenolol, practolol, procinolol, exaprolol, cicloprolol, carazolol, tazolol, tienoxolol, oxprenolol, propranolol, IPS 339, labetolol, dilevalol, esmolol, bupranolol, bunolol, isoxaprolol, diacetolol, hydroxylevobunolol, carvedilol and the like. The preferred beta-blocker is betaxolol, especially S-betaxolol.

Other preferred beta-blockers are certain 4-(3-substituted amino-2-hydroxypropoxy)-1,2,5-thiadiazoles which were originally disclosed in German Patent No. 1,925,956 (issued in 1969 to B. K. Wasson), equivalent to US 3,655,663 (issued in 1972) and US 3,729,469 (issued in 1973). These thiadiazoles have the following general structure: 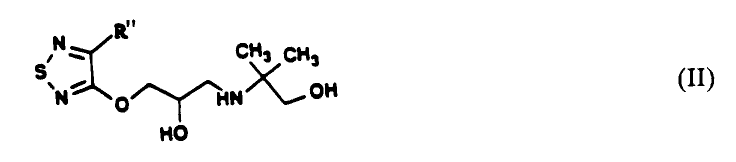 and optically active isomers and pharmacologically acceptable salts thereof, wherein R'' represents: (1) hydrogen; (2) halogen, preferably chloro or bromo; (3) C₁₋₅ lower alkyl having either a straight or branched chain such as methyl, ethyl, propyl, isopropyl, butyl iso-, secondary- or tert-butyl and amyl, including all of its branched chain configurations; (4) C₂₋₅ lower alkenyl, such as vinyl, allyl, methallyl and the like; (5) a group having the structure Y-X-Z-, wherein Y is either a straight or branched chain C₁₋₄ alkyl optionally substituted with a phenyl group or a phenyl optionally substituted with one or more halogen atoms (especially chloro, bromo, fluoro), hydroxy, C₁₋₃ lower alkyl or alkoxy, X is oxygen or sulfur and Z is a C₁₋₂ alkyl; (6) a carbamoyl group having the structure R''₁HNCO, wherein R''₁ is a C₁₋₅ lower alkyl; (7) C₃₋₆ cycloalkyl, such as cyclopropyl, cyclobutyl, cyclopentyl, cyclohexyl and the like; (8) C₁₋₅ lower alkoxy, either a straight or branched chain and including methoxy, ethoxy, propoxy, isopropoxy, butoxy, and pentoxy (the latter groups existing in either straight or branched configuration); (9) phenyl or substituted phenyl, wherein the substitutes are selected from one or more halogen atoms (preferably chloro or fluoro) and a C₁₋₃ lower alkyl or alkoxy; (10) phenyl-lower alkyl, wherein the lower alkyl moiety is either a straight or branched chain and has from 1 to 4 carbons and the phenyl moiety can be unsubstituted or substituted with one or more halogen atoms (preferably chloro, fluoro, or bromo) or C₁₋₃ lower alkyl or alkoxy; (11) an amino having the structure - NR''₂R''₃, wherein R''₂ represents hydrogen, C₁₋₄ lower alkyl and C₂₋₄ hydroxy-substituted lower alkyl, R''₃ represents hydrogen, C₁₋₄ lower alkyl, a hydroxy-substituted lower alkyl and phenyl, or R''₂ and R''₃ can be joined together either directly to give a 3 to 7 membered ring with the nitrogen to which they are attached thereby forming aziridinyl, azetidinyl, pyrrolidyl, piperidyl, or a hexahydroazepinyl group, said 3 to 7 membered rings being either unsubstituted or substituted, preferably with one or more C₁₋₅ lower alkyl and C₁₋₃ hydroxy-lower alkyl, or alternatively R''₂ and R''₃ can be joined through an oxygen, nitrogen or sulfur atom to form a 5 or 6 membered ring, advantageously a morpholino, hexahydropyrimidyl, thiazolidinyl, p-thiazinyl, piperazinyl and the like group optionally substituted by C₁₋₃ lower alkyl; or (12) R additionally can be a 5 or 6 membered heterocyclic ring having oxygen, nitrogen or sulfur as the hetero atom and preferably the 2-furyl, 2- or 3-thienyl, 2-pyrryl and the o-, m- or p-pyridyl. These thiadiazoles may be prepared by the methods disclosed in US 3,655,663 and US 3,729,469 whose entire contents are incorporated by reference herein. Especially preferred thiadiazoles are those of Structure (II), above, wherein R'' is chloro, ethyl, allyl, cyclopropyl, ethoxy, phenyl, phenyl-chloromethyl, or 2-(cyclopropylmethoxy)ethyl.

The CAIs which are used in the compositions of the present invention are those disclosed in U.S. Patent Application Serial No. 07/755,313. Such CAIs have the following generic structure: 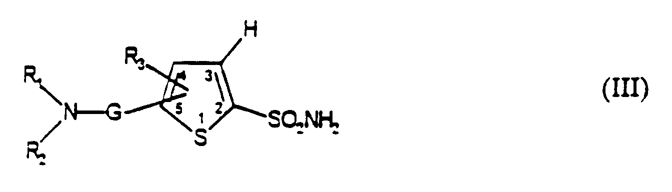 or a pharmaceutically acceptable salt thereof, wherein:
- R₁ is:: H; C₁₋₄ alkyl; C₂₋₄ alkyl substituted optionally with OH, halogen, C₁₋₄ alkoxy or C(=O)R₇;
- R₂ is:: H; C₁₋₈ alkyl; C₂₋₈ alkyl substituted with OH, NR₅R₆, halogen, C₁₋₄ alkoxy, C₂₋₄alkoxyC₁₋₄alkoxy, OC(=O)R₇, or C(=O)R₇; C₃₋₇ alkenyl unsubstituted or substituted optionally with OH, NR₅R₆, or C₁₋₄ alkoxy; C₃₋₇ alkynyl unsubstituted or substituted optionally with OH, NR₅R₆, or C₁₋₄ alkoxy, C₁₋₃ alkyl substituted with phenyl or R₁₀ either of which can be unsubstituted or substituted optionally with C₁₋₃ alkyl, C₁₋₃ haloalkyl, OH, (CH₂)ₙNR₅R₆, halogen, C₁₋₄ alkoxy, C₁₋₄ haloalkoxy, C(=O)R₇, S(=O)ₘR₈ or SO₂NR₅R₆, wherein m is 0 - 2 and n is 0 - 2; C₂₋₄ alkoxy substituted optionally with NR₅R₆, halogen, C₁₋₄ alkoxy, or C(=O)R₇; phenyl or R₁₀ either of which can be unsubstituted or substituted optionally with C₁₋₃ alkyl, C₁₋₃ haloalkyl, OH, (CH₂)ₙNR₅R₆, halogen, C₁₋₄ alkoxy, C₁₋₄ haloalkoxy, C(=O)R₇, S(=O)ₘR₈ or SO₂NR₅R₆, wherein m is 0 - 2 and n is 0 - 2; provided that R₁ and R₂ cannot both be H; or R₁ and R₂ can be joined to form a saturated ring of 5 or 6 atoms selected from O, S, C or N, such as, pyrrolidine, oxazolidine, thiomorpholine, thiomorpholine 1,1 dioxide, morpholine, piperazine, thiazolidine 1,1 dioxide, or tetrahydrooxazine, which can be unsubstituted or substituted optionally on carbon with OH, NR₅R₆, halogen, C₁₋₄ alkoxy, C(=O)R₇, C₁₋₆ alkyl, C₁₋₆ alkyl substituted optionally with OH, NR₅R₆, halogen, C₁₋₄ alkoxy, C(=O)R₇ or on nitrogen with NR₅R₆, C₁₋₄ alkoxy, C(=O)R₇, C₁₋₆ alkyl or C₂₋₆ alkyl, substituted optionally with OH, NR₅R₆, halogen, C₁₋₄ alkoxy or C(=O)R₇;
- R₃ is:: H; halogen; C₁₋₄ alkyl; C₁₋₈ alkoxy; C₁₋₈ alkylthiol; C₂₋₈ alkoxy substituted optionally with OH, NR₅R₆, halogen, C₁₋₄ alkoxy or C(=O)R₇; C₁₋₄ alkyl substituted optionally with R₄; or R₁ and R₃ can be joined together with carbon atoms to form a ring of from 5 to 7 members in which said carbon atoms can be unsubstituted or substituted optionally with R₄;
- R₄ is:: OH; C₁₋₄ alkyl unsubstituted or substituted optionally with OH, NR₅R₆, halogen, C₁₋₄ alkoxy or C(=O)R₇; C₁₋₄ alkoxy; C₂₋₄ alkoxy substituted optionally with OH, NR₅R₆, halogen, C₁₋₄ alkoxy or C(=O)R₇; NR₅R₆; phenyl or R₁₀ either of which can be unsubstituted or substituted optionally with OH, (CH₂)ₙNR₅R₆, halogen, C₁₋₄ alkoxy, C₁₋₄ haloalkoxy, C(=O)R₇, S(=O)ₘR₈ or SO₂NR₅R₆, wherein m is 0 - 2 and n is 0 - 2;

Provided that when G is SO₂ and R₃ is in the 4 position and is H or halogen then R₁ and R₂ are not H, C₁₋₆ alkyl substituted optionally with OH, C₁₋₆ alkoxy, C₂₋₆ alkoxycarbonyl, C₂₋₆ alkenyl, phenyl, phenoxy, pyridyl, tetrahydrofuryl, C₂₋₆ alkanoyl, C₂₋₆ alkenyl, nor are they joined to form a 5, 6 or 7 member ring, saturated or unsaturated, comprised of atoms selected optionally from C, O, S, N in which said nitrogen, when saturated is substituted optionally with H or C₁₋₆ alkyl or in which said carbon is substituted optionally with C₁₋₆ alkyl, C₁₋₆ alkoxy or OH; and when R₃ is in the 5 position and is H, Cl, Br, or C₁₋₃ alkyl then neither R₁ nor R₂ can be H or C₁₋₄ alkyl; and when G is C(=O) and in the 5- position and R₃ is H, then R₁ and R₂ cannot both be CH₃;
- R₅ & R₆: are the same or different and are: H; C₁₋₄ alkyl; C₂₋₄ alkyl substituted optionally with OH, halogen, C₁₋₄ alkoxy or C(=O)R₇; C₁₋₄ alkoxy; C₂₋₄ alkoxy substituted optionally with OH, halogen, C₁₋₄ alkoxy or C(=O)R₇; C₃₋₇ alkenyl unsubstituted or substituted optionally with OH, NR₅R₆, or C₁₋₄ alkoxy; C₃₋₇ alkynyl unsubstituted or substituted optionally with OH, NR₅R₆, or C₁₋₄ alkoxy; C₁₋₂alkyl-C₃₋₅cycloalkyl: C(=O)R₇ or R₅ and R₆ can be joined to form a ring of 5 or 6 atoms selected from O, S, C or N, such as, pyrrolidine, oxazolidine, thiomorpholine, thiomorpholine 1,1 dioxide, morpholine, piperazine or thiazolidine 1,1-dioxide, which can be unsubstituted or substituted optionally on carbon with OH, (=O), halogen, C₁₋₄ alkoxy, C(=O)R₇, C₁₋₆ alkyl, C₁₋₆ alkyl substituted optionally with OH, halogen, C₁₋₄ alkoxy, C(=O)R₇ or on nitrogen with C₁₋₄ alkoxy, C(=O)R₇, S(=O)ₘR₈, C₁₋₆ alkyl or C₂₋₆ alkyl substituted optionally with OH, halogen, C₁₋₄ alkoxy, C(=O)R₇ or on sulfur by (=O)ₘ, wherein m is 0 - 2;
- R₇ is:: C₁₋₈ alkyl; C₁₋₈ alkyl substituted optionally with OH, NR₅R₆, halogen, C₁₋₄ alkoxy or C(=O)R₉; C₁₋₄ alkoxy; C₂₋₄ alkoxy substituted optionally with OH, NR₅R₆, halogen or C₁₋₄ alkoxy; NR₅R₆; or phenyl or R₁₀ either of which can be unsubstituted or substituted optionally with OH, halogen, C₁₋₃ alkyl, C₁₋₃ haloalkoxy, (CH₂)ₙNR₅R₆, S(=O)ₘR₈ or SO₂NR₅R₆, wherein n is 0 or 1 and m is 0-2;
- R₈ is:: C₁₋₄ alkyl; C₂₋₄ alkyl substituted optionally with OH, NR₅R₆, halogen, C₁₋₄ alkoxy or C(=O)R₇;
- R₉ is:: C₁₋₄ alkyl; C₁₋₄ alkoxy; amino, C₁₋₃ alkylamino, or di-C₁₋₃ alkylamino;
- R₁₀ is:: a monocyclic ring system of 5 or 6 atoms composed of C, N, O, and/or S, such as furan, thiophene, pyrrole, pyrazole, imidazole, triazole, tetrazole, oxazole, isoxazole, isothiazole, thiazole, thiadiazole, pyridine, pyrimidine, pyridazine, and pyrazine; and
- G is:: C(=O) or SO₂.

The CAI can further be (-)-trans-5,6-dihydro-6-(3-methoxy)propyl-4-propylamino-4H-thieno-[2,3-b]thiopyran-2-sulfonamide 7,7-dioxide.

In the above definitions, the total number of carbon atoms in a substituent group is indicated by the Cᵢ₋ⱼ prefix where i and j are numbers from 1 to 8 for example. This Cᵢ₋ⱼ definition includes both the straight and branched chain isomers. For example, C₁₋₄ alkyl would designate methyl through the butyl isomers; and C₁₋₄ alkoxy would designate methoxy through the butoxy isomers.

The term "halogen," either alone or in compound words such as "haloalkyl," means fluorine, chlorine, bromine or iodine. Further, when used in compound words such as "haloalkyl," said alkyl may be partially or fully substituted with halogen atoms, which may be the same or different.

Structure (III) includes isomers, wherein R₃ and GNR₁R₂ are attached to the 4 and 5 position respectively or R₃ is attached to the 5 position and GNR₁R₂ is attached to the 4 position. Many of the novel compounds of Structure (III) possess one or more chiral centers and this invention includes all enantiomers, diastereomers and mixtures thereof.

Especially preferred CAIs of the present invention are those listed in Table 1, below.

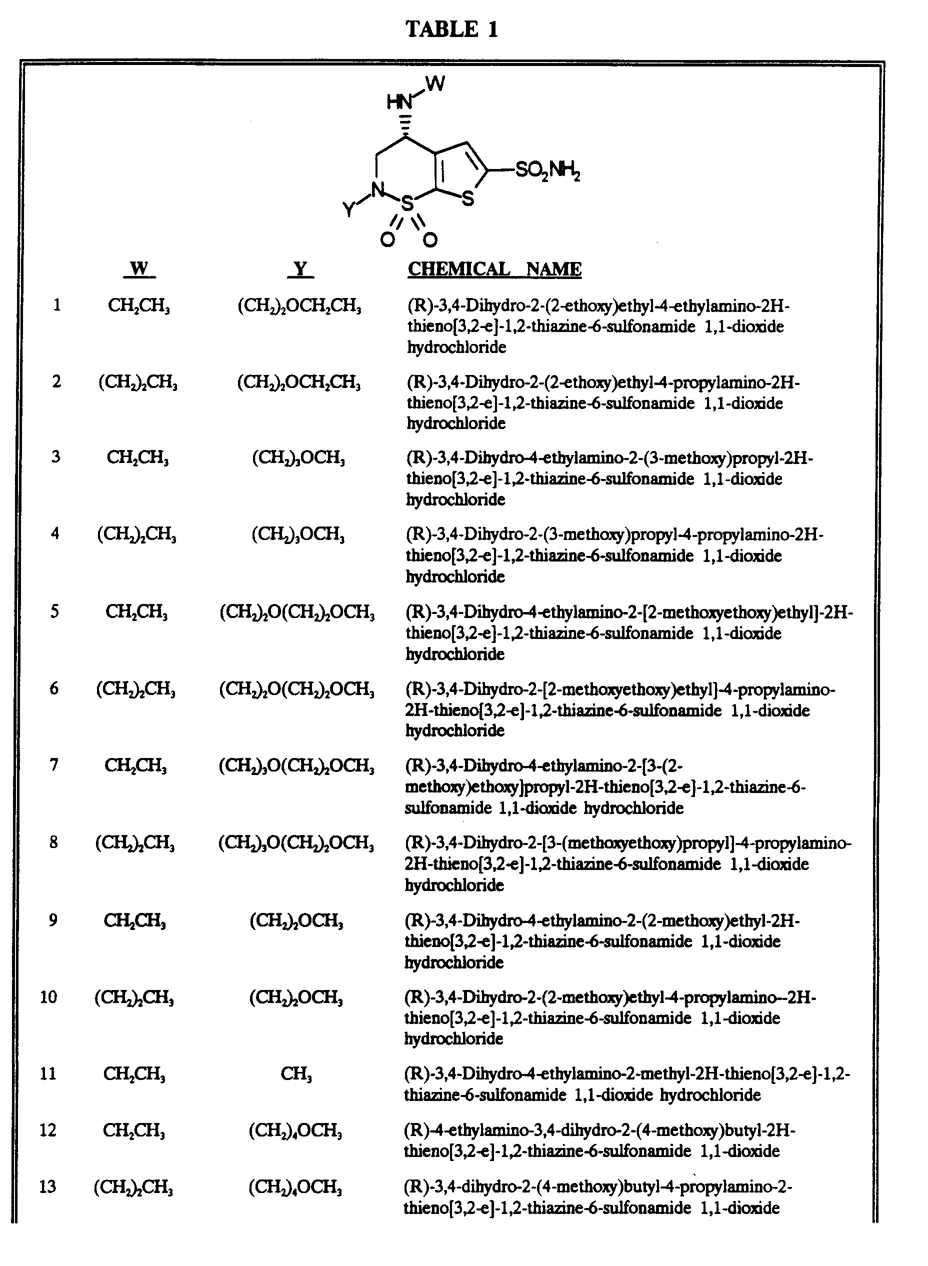

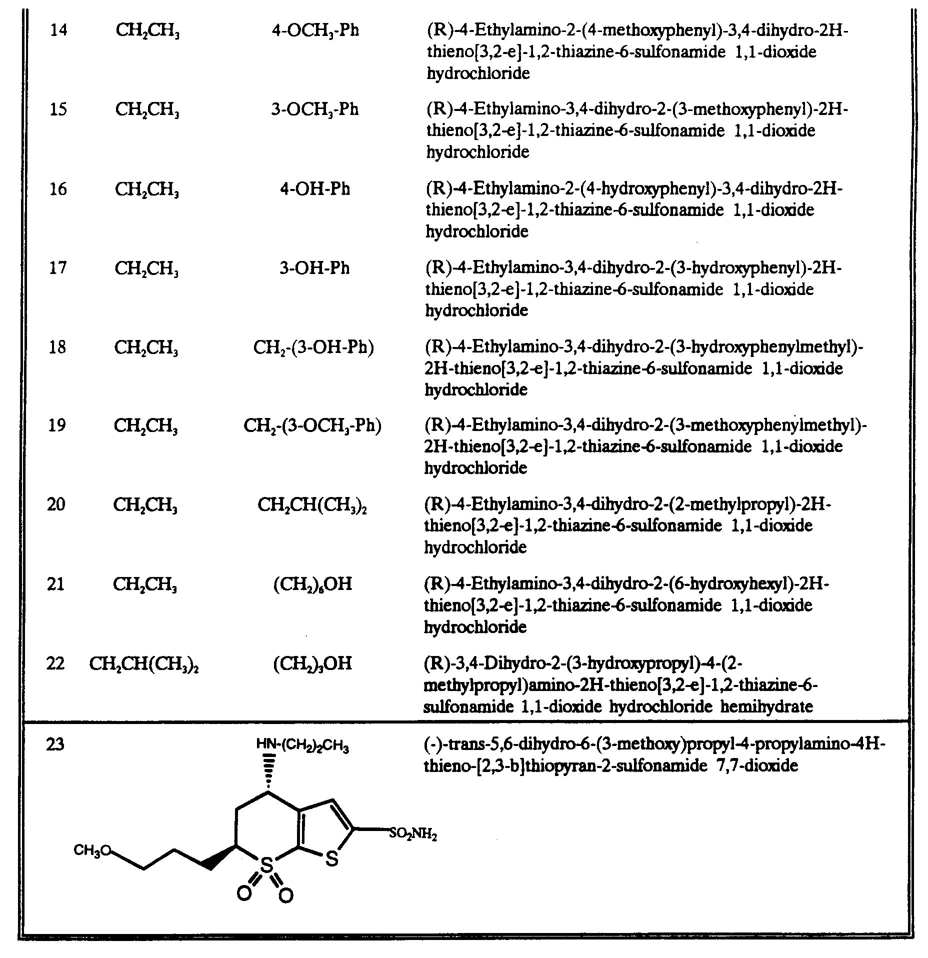

In general, an amount of a beta-blocker less than or equal to about 2.0% by weight (wt%) and amount of a CAI less than or equal to about 5 wt% are used. It is preferred that an amount of beta-blocker between about 0.01 and about 1.0 wt% is used and it is especially preferred to use an amount between about 0.05 to about 0.5 wt%. An amount of a CAI between about 0.25 and about 3 wt% is preferred and an amount between about 0.5 and about 2 wt% is especially preferred. The ratio by weight of beta-blocker to CAI is generally between about 4:1 to about 1:300, preferably between about 1:1 to about 1:40.

The high molecular weight, anionic mucomimetic polymers useful in the present invention have a molecular weight between about 50,000 and 6 million daltons. The polymers are characterized as having carboxylic acid functional groups and preferably contain between 2 and 7 carbon atoms per functional group. The gels which form during preparation of the ophthalmic polymer dispersion have a viscosity between about 1,000 to about 300,000 centipoise (cps). Suitable polymers are carboxy vinyl polymers, preferably those called Carbomers, e.g., Carbopol^{•} (B.F. Goodrich Co., Cleveland, Ohio). Specifically preferred are Carbopol^{•} 934 and 940. Such polymers will typically be employed in an amount between about 0.05 and about 8.0 wt%, depending on the desired viscosity of the composition. Pourable liquid compositions generally comprise an amount of the polymer between about 0.05 and about 2.0 wt%.

The DCS component of the present compositions is added to provide an additional means of controlling release, as well as to prevent the stinging which often occurs with the topical administration of certain drugs, such as betaxolol. As used herein, the term "finely-divided drug carrier substrate" (or "DCS") means finely-divided solids, colloidal particles, or soluble polymers and/or polyelectrolytes which are capable of selective adsorption or binding with drug molecules. Examples of DCS include, but are not limited to: finely divided silica, such as fumed silica, silicates and bentonites; ion exchange resins, which can be anionic, cationic or non-ionic in nature; and soluble polymers, such as, alginic acid, pectin, soluble carrageenans, Carbopol®, and polystyrene sulfonic acid. Preferred DCS are the ion exchange resins. Some resins which are used in chromatography make ideal DCS for binding drugs in the compositions of the present invention. The DCS component is present in the compositions of the present invention at a concentration between about 0.05 and about 10.0% by weight.

The size of the DCS can be important, both with respect to mode of action and comfort. The average particle size of the typical commercially available form of the DCS material of choice, an ion exchange resin, is about 40 to about 150 microns. Such particles are most conveniently reduced to a particle size range of about 1.0 to about 25.0 microns, preferably between about 1.0 and 10.0 microns, by ball milling, according to known techniques. In the alternative, small particles may be synthesized in the optimal size range of 3-7 microns. Although this procedure can be more expensive, it is superior in providing a more uniform and narrow distribution of sizes in the preferred range.

These anionic mucomimetic polymers and DCS are discussed in greater detail in U.S. 4,911,920 issued 27 March 1990 and EP 507 224 (published 7 October 1992). The entire contents of the patent and patent application are hereby incorporated by reference herein.

In addition to the above-described principal ingredients, the anti-glaucoma compositions of the present invention may further comprise various formulatory ingredients, such as antimicrobial preservatives and tonicity agents. Examples of suitable antimicrobial preservatives include: benzalkonium chloride, thimerosal, chlorobutanol, methyl paraben, propyl paraben, phenylethyl alcohol, edetate disodium, sorbic acid, Onamer M® and other agents equally well-known to those skilled in the art. Such preservatives, if utilized, will typically be employed in an amount between about 0.001 to 1.0 wt%. Examples of suitable agents which may be utilized to adjust the tonicity or osmolality of the formulations include: sodium chloride, potassium chloride, mannitol, dextrose, glycerin and propylene glycol. Such agents, if utilized, will typically be employed in an amount between about 0.1 to 10.0 wt%.

As will be appreciated by those skilled in the art, the compositions may be formulated in various dosage forms suitable for topical ophthalmic delivery, including solutions, suspensions, emulsions, gels and erodible solid ocular inserts. The compositions preferably are aqueous, have a pH between 5.0 to 7.8 and an osmolality between 280 to 320 milliOsmoles per kilogram (mOsm/kg).

The following example further illustrates the anti-glaucoma compositions of the present invention.

### Example 1

The following formulations are typical of aqueous ophthalmic suspensions of the present invention.

| **INGREDIENT** | **AMOUNT (wt%)** | | | | | |
|---|---|---|---|---|---|---|
| | **A** | **B** | **C** | **D** | **E** | **F** |
| Betaxolol HCl | 0.28 | --- | 0.28 | --- | 0.28 | --- |
| Compound 3* | 1.7** | 1.7** | --- | --- | --- | --- |
| Compound 12* | --- | --- | 1.5 | 1.5 | --- | --- |
| Compound 13* | --- | --- | --- | --- | 1.5 | 1.5 |
| Timolol maleate | --- | 0.68 | --- | 0.68 | --- | 0.68 |
| BAC | 0.01 | 0.01 | 0.01 | 0.01 | 0.01 | 0.01 |
| EDTA | 0.05 | 0.05 | 0.05 | 0.05 | 0.05 | 0.05 |
| Carbopol^{•} 934P | 0.4 | 0.4 | 0.4 | 0.4 | 0.4 | 0.4 |
| Polysorbate 80 | 0.05 | 0.05 | 0.05 | 0.05 | 0.05 | 0.05 |
| Mannitol | qs to 300 mOsm/kg | qs to 300 mOsm/kg | qs to 300 mOsm/kg | qs to 300 mOsm/kg | qs to 300 mOsm/kg | qs to 300 mOsm/kg |
| pH | qs to 7.5 | qs to 7.5 | qs to 7.5 | qs to 7.5 | qs to 7.5 | qs to 7.5 |
| Water | qs to 100 | qs to 100 | qs to 100 | qs to 100 | qs to 100 | qs to 100 |

| | | | | | | |
|---|---|---|---|---|---|---|
| *See Table 1. | | | | | | |
| **Roughly equivalent to 1.5 wt% of the free base. | | | | | | |

### Preparation:

Compound 3, 12 or 13, and betaxolol or timolol are mixed in 50% of the total water volume component to form an uniform dispersion. Carbopol 934P is slowly added as an aqueous dispersion. The mixture is then homogenized at high speed. The other ingredients are added as aqueous solutions and then water is added to make the final volume. The resultant products, A-F, will be white uniform suspensions.

### Example 2

The following formulations are typical of aqueous ophthalmic suspensions of the present invention.

| **INGREDIENT** | **AMOUNT (wt%)** | | | | | | | |
|---|---|---|---|---|---|---|---|---|
| | **G** | **H** | **J** | **K** | **L** | **M** | **N** | **O** |
| Betaxolol HCl | 0.28 | 0.56 | 0.28 | 0.56 | 0.28 | 0.56 | 0.28 | 0.56 |
| Compound 3* | --- | --- | 1.7** | 1.7** | --- | --- | --- | --- |
| Compound 9* | 1.67** | 1.67** | --- | --- | 1.67** | 1.67** | 1.67** | 1.67** |
| Compound 12* | --- | --- | --- | --- | 1.5 | 1.5 | --- | --- |
| Compound 13* | --- | --- | --- | --- | --- | --- | 1.5 | 1.5 |
| BAC | 0.01 | 0.01 | 0.01 | 0.01 | 0.01 | 0.01 | 0.01 | 0.01 |
| EDTA | 0.05 | 0.05 | 0.05 | 0.05 | 0.05 | 0.05 | 0.05 | 0.05 |
| Amberlite^{•} IRP-69 | 0.25 | 0.50 | 0.25 | 0.50 | 0.25 | 0.50 | 0.25 | 0.50 |
| Carbopol^{•} 934P | 0.4 | 2.0 | 0.4 | 2.0 | 0.4 | 2.0 | 0.4 | 2.0 |
| Polysorbate 80 | 0.05 | 0.05 | 0.05 | 0.05 | 0.05 | 0.05 | 0.05 | 0.05 |
| Mannitol | qs to 300 mOsm/kg | qs to 300 mOsm/kg | qs to 300 mOsm/kg | qs to 300 mOsm/kg | qs to 300 mOsm/kg | qs to 300 mOsm/kg | qs to 300 mOsm/kg | qs to 300 mOsm/kg |
| pH | qs to 7.5 | qs to 7.5 | qs to 7.5 | qs to 7.5 | qs to 7.5 | qs to 7.5 | qs to 7.5 | qs to 7.5 |
| Water | qs to 100 | qs to 100 | qs to 100 | qs to 100 | qs to 100 | qs to 100 | qs to 100 | qs to 100 |

| | | | | | | | | |
|---|---|---|---|---|---|---|---|---|
| *SeeTable 1. | | | | | | | | |
| **Roughly equivalent to 1.5 wt% of the free base. | | | | | | | | |

### Preparation:

Amberlite, betaxolol and Compound 3, 9, 12 or 13 are mixed in 50% of the total water volume component to form an uniform dispersion. Carbopol 934P is slowly added as an aqueous dispersion. The mixture is then homogenized at high speed. The other ingredients are added as aqueous solutions and then water is added to make the final volume. The resultant products, G-O, will be white uniform suspensions.

The present invention is also directed to methods of treating and controlling ocular hypertension associated with glaucoma and other ophthalmic diseases and abnormalities. The methods comprise topically applying to the affected eye(s) of the patient a therapeutically effective amount of a composition according to the present invention. The frequency and amount of dosage will be determined by the clinician based on various clinical factors. The methods will typically comprise topical application of one or two drops (or an equivalent amount of a solid or semi-solid dosage form) to the affected eye one to two times per day.

The invention has been described by reference to certain preferred embodiments; however, it should be understood that it may be embodied in other specific forms or variations thereof without departing from its spirit or essential characteristics. The embodiments described above are therefore considered to be illustrative in all respects and not restrictive, the scope of the invention being indicated by the appended claims rather than by the foregoing description.

## Claims

1. A topical ophthalmic composition, comprising a beta-blocker and a carbonic anhydrase inhibitor in an ophthalmically acceptable vehicle, wherein the composition is a suspension and the final composition pH is between about 5.0 and about 7.8 and the carbonic anhydrase inhibitor has the formula: 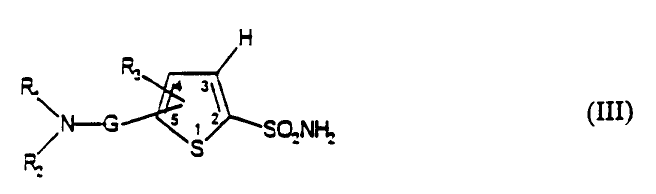 or a pharmaceutically acceptable salt thereof, wherein:
R₁ is: H; C₁₋₄ alkyl; C₂₋₄ alkyl substituted optionally with OH, halogen, C₁₋₄ alkoxy or C(=O)R₇;
R₂ is: H; C₁₋₈ alkyl; C₂₋₈ alkyl substituted with OH, NR₅R₆, halogen, C₁₋₄ alkoxy, C₂₋₄alkoxyC₁₋₄alkoxy, OC(=O)R₇, or C(=O)R₇; C₃₋₇ alkenyl unsubstituted or substituted optionally with OH, NR₅R₆, or C₁₋₄ alkoxy; C₃₋₇ alkynyl unsubstituted or substituted optionally with OH, NR₅R₆, or C₁₋₄ alkoxy; C₁₋₃ alkyl substituted with phenyl or R₁₀ either of which can be unsubstituted or substituted optionally with C₁₋₃ alkyl, C₁₋₃ haloalkyl, OH, (CH₂)ₙNR₅R₆, halogen, C₁₋₄ alkoxy, C₁₋₄ haloalkoxy, C(=O)R₇, S(=O)ₘR₈ or SO₂NR₅R₆, wherein m is 0 - 2 and n is 0 - 2; C₂₋₄ alkoxy substituted optionally with NR₅R₆, halogen, C₁₋₄ alkoxy, or C(=O)R₇; phenyl or R₁₀ either of which can be unsubstituted or substituted optionally with C₁₋₃ alkyl, C₁₋₃ haloalkyl, OH, (CH₂)ₙNR₅R₆, halogen, C₁₋₄ alkoxy, C₁₋₄ haloalkoxy, C(=O)R₇, S(=O)ₘR₈ or SO₂NR₅R₆, wherein m is 0 - 2 and n is 0 - 2; provided that R₁ and R₂ cannot both be H; or R₁ and R₂ can be joined to form a saturated ring of 5 or 6 atoms selected from O, S, C or N, such as, pyrrolidine, oxazolidine, thiomorpholine, thiomorpholine, 1,1 dioxide, morpholine, piperazine, thiazolidine 1,1 dioxide, or tetrahydrooxazine, which can be unsubstituted or substituted optionally on carbon with OH, NR₅R₆, halogen, C₁₋₄ alkoxy, C(=O)R₇, C₁₋₆ alkyl, C₁₋₆ alkyl substituted optionally with OH, NR₅R₆, halogen, C₁₋₄ alkoxy, C(=O)R₇ or on nitrogen with NR₅R₆, C₁₋₄ alkoxy, C(=O)R₇, C₁₋₆ alkyl or C₂₋₆ alkyl substituted optionally with OH, NR₅R₆, ,halogen C₁₋₄ alkoxy or C(=O)R₇;
R₃ is: H; halogen; C₁₋₄ alkyl; C₁₋₈ alkoxy; C₁₋₈ alkylthiol; C₂₋₈ alkoxy substituted optionally with OH, NR₅R₆, halogen, C₁₋₄ alkoxy or C(=O)R₇; C₁₋₄ alkyl substituted optionally with R₄; or R₁ and R₃ can be joined together with carbon atoms to form a ring of from 5 to 7 members in which said carbon atoms can be unsubstituted or substituted optionally with R₄;
R₄ is: OH; C₁₋₄ alkyl unsubstituted or substituted optionally with OH, NR₅R₆, halogen, C₁₋₄ alkoxy, or C(=O)R₇; C₁₋₄ alkoxy; C₂₋₄ alkoxy substituted optionally with OH, NR₅R₆, halogen, C₁₋₄ alkoxy or C(=O)R₇; NR₅R₆; phenyl or R₁₀ either of which can be unsubstituted or substituted optionally with OH, (CH₂)ₙNR₅R₆, halogen, C₁₋₄ alkoxy, C₁₋₄ haloalkoxy, C(=O)R₇, S(=O)ₘR₈ or SO₂NR₅R₆, wherein m is 0 - 2 and n is 0 - 2;
Provided that when G is SO₂ and R₃ is in the 4 position and is H or halogen then R₁ and R₂ are not H, C₁₋₆ alkyl substituted optionally with OH, C₁₋₆ alkoxy, C₂₋₆ alkoxycarbonyl, C₂₋₆ alkenyl, phenyl, phenoxy, pyridyl, tetrahydrofuryl, C₂₋₆ alkanoyl, C₂₋₆ alkenyl, nor are they joined to form a 5, 6 or 7 member ring, saturated or unsaturated, comprised of atoms selected optionally from C, O, S, N in which said nitrogen when saturated, is substituted optionally with H or C₁₋₆ alkyl or in which said carbon is substituted optionally with C₁₋₆ alkyl, C₁₋₆ alkoxy or OH; and when R₃ is in the 5 position and is H, Cl, Br, or C₁₋₃ alkyl then neither R₁ nor R₂ can be H or C₁₋₄ alkyl; and when G is C(=O) and in the 5- position and R₃ is H, then R₁ and R₂ cannot both be CH₃;
R₅ & R₆ are the same or different and are: H; C₁₋₄ alkyl; C₂₋₄ alkyl substituted optionally with OH, halogen, C₁₋₄ alkoxy or C(=O)R₇; C₁₋₄ alkoxy; C₂₋₄ alkoxy substituted optionally with OH, halogen, C₁₋₄ alkoxy or C(=O)R₇; C₃₋₇ alkenyl unsubstituted or substituted optionally with OH, NR₅R₆, or C₁₋₄ alkoxy; C₃₋₇ alkynyl unsubstituted or substituted optionally with OH, NR₅R₆, or C₁₋₄ alkoxy; C₁₋₂alkyl-C₃₋₅cycloalkyl; C(=O)R₇ or R₅ and R₆ can be joined to form a ring of 5 or 6 atoms selected from O, S, C or N, such as, pyrrolidine, oxazolidine, thiomorpholine, thiomorpholine 1,1 dioxide, morpholine, piperazine or thiazolidine 1,1-dioxide, which can be unsubstituted or substituted optionally on carbon with OH, (=O), halogen, C₁₋₄ alkoxy, C(=O)R₇, C₁₋₆ alkyl, C₁₋₆ alkyl substituted optionally with OH, halogen, C₁₋₄ alkoxy, C(=O)R₇ or on nitrogen with C₁₋₄ alkoxy, C(=O)R₇, S(=O)ₘR₈, C₁₋₆ alkyl or C₂₋₆ alkyl substituted optionally with OH, halogen, C₁₋₄ alkoxy, C(=O)R₇ or on sulfur by (=O)ₘ, wherein m is 0 - 2;
R₇ is: C₁₋₈ alkyl; C₁₋₈ alkyl substituted optionally with OH, NR₅R₆, halogen, C₁₋₄ alkoxy or C(=O)R₉; C₁₋₄ alkoxy; C₂₋₄ alkoxy substituted optionally with OH, NR₅R₆, halogen or C₁₋₄ alkoxy; NR₅R₆; or phenyl or R₁₀ either of which can be substituted or substituted optionally with OH, halogen, C₁₋₃ alkyl, C₁₋₃ haloalkoxy, (CH₂)ₙNR₅R₆, S(=O)ₘR₈ or SO₂NR₅R₆, wherein n is 0 or 1 and m is 0-2;
R₈ is: C₁₋₄ alkyl; C₂₋₄ alkyl substituted optionally with OH, NR₅R₆, halogen, C₁₋₄ alkoxy or C(=O)R₇;
R₉ is: C₁₋₄ alkyl; C₁₋₄ alkoxy; amino, C₁₋₃ alkylamino, or di-C₁₋₃ alkylamino;
R₁₀ is: a monocyclic ring system of 5 or 6 atoms composed of C, N, O, and/or S, such as furan, thiophene, pyrrole, pyrazole, imidazole, triazole, tetrazole, oxazole, isoxazole, isothiazole, thiazole, thiadiazole, pyridine, pyrimidine, pyridazine, and pyrazine; and
G is: C (=O) or SO₂ or
wherein the carbonic anhydrase inhibitor is (-)-trans-5,6-dihydro-6-(3-methoxy)propyl-4-propylamino-4H-thieno[2,3-b]thiopyran-2-sulfonamide 7,7-dioxide.

2. The composition of claim 1, wherein the carbonic anhydrase inhibitor is selected from the group consisting of: (R)-3,4-Dihydro-2-(2-ethoxy)ethyl-4-ethylamino-2H-thieno[3,2-e]-1,2-thiazine-6-sulfonamide 1,1-dioxide; (R)-3,4-Dihydro-2-(2-ethoxy)ethyl-4-propylamino-2H-thieno[3,2-e]-1,2-thiazine-6-sulfonamide 1,1-dioxide; (R)-3,4-Dihydro-4-ethylamino-2-(3-methoxy)propyl-2H-thieno[3,2-e]-1,2-thiazine-6-sulfonamide 1,1-dioxide; (R)-3,4-Dihydro-2-(3-methoxy)propyl-4-propylamino-2H-thieno[3,2-e]-1,2-thiazine-6-sulfonamide 1,1-dioxide; (R)-3,4-Dihydro-4-ethylamino-2-[2-methoxyethoxy)ethyl]-2H-thieno[3,2-e]-1,2-thiazine-6-sulfonamide 1,1-dioxide; (R)-3,4-Dihydro-2-[2-methoxyethoxy)ethyl]-4-propylamino-2H-thieno[3,2-e]-1,2-thiazine-6-sulfonamide 1,1-dioxide; (R)-3,4-Dihydro-4-ethylamino-2-[3-(2-methoxy)ethoxy]propyl-2H-thieno[3,2-e]-1,2-thiazine-6-sulfonamide 1,1-dioxide; (R)-3,4-Dihydro-2-[3-(methoxyethoxy)propyl]-4-propylamino-2H-thieno[3,2-e]-1,2-thiazine-6-sulfonamide 1,1-dioxide; (R)-3,4-Dihydro-4-ethylamino-2-(2-methoxy)ethyl-2H-thieno[3,2-e]-1,2-thiazine-6-sulfonamide 1,1-dioxide; (R)-3,4-Dihydro-2-(2-methoxy)ethyl-4-propylamino--2H-thieno[3,2-e]-1,2-thiazine-6-sulfonamide 1,1-dioxide; (R)-3,4-Dihydro-4-ethylamino-2-methyl-2H-thieno[3,2-e]-1,2-thiazine-6-sulfonamide 1,1-dioxide; (R)-4-ethylamino-3,4-dihydro-2-(4-methoxy)butyl-2H-thieno[3,2-e]-1,2-thiazine-6-sulfonamide 1,1-dioxide; (R)-3,4-dihydro-2-(4-methoxy)butyl-4-propylamino-2-thieno[3,2-e]-1,2-thiazine-6-sulfonamide 1,1-dioxide; (R)-4-Ethylamino-2-(4-methoxyphenyl)-3,4-dihydro-2H-thieno[3,2-e]-1,2-thiazine-6-sulfonamide 1,1-dioxide; (R)-4-Ethylamino-3,4-dihydro-2-(3-methoxyphenyl)-2H-thieno[3,2-e]-1,2-thiazine-6-sulfonamide 1,1-dioxide; (R)-4-Ethylamino-2-(4-hydroxyphenyl)-3,4-dihydro-2H-thieno[3,2-e]-1,2-thiazine-6-sulfonamide 1,1-dioxide; (R)-4-Ethylamino-3,4-dihydro-2-(3-hydroxyphenyl)-2H-thieno[3,2-e]-1,2-thiazine-6-sulfonamide 1,1-dioxide; (R)-4-Ethylamino-3,4-dihydro-2-(3-hydroxyphenylmethyl)-2H-thieno[3,2-e]-1,2-thiazine-6-sulfonamide 1,1-dioxide; (R)-4-Ethylamino-3,4-dihydro-2-(3-methoxyphenylmethyl)-2H-thieno[3,2-e]-1,2-thiazine-6-sulfonamide 1,1-dioxide; (R)-4-Ethylamino-3,4-dihydro-2-(2-methylpropyl)-2H-thieno[3,2-e]-1,2-thiazine-6-sulfonamide 1,1-dioxide; (R)-4-Ethylamino-3,4-dihydro-2-(6-hydroxyhexyl)-2H-thieno[3,2-e]-1,2-thiazine-6-sulfonamide 1,1-dioxide; (R)-3,4-Dihydro-2-(3-hydroxypropyl)-4-(2-methylpropyl)amino-2H-thieno[3,2-e]-1,2-thiazine-6-sulfonamide 1,1-dioxide; and (-)-trans-5,6-dihydro-6-(3-methoxy)propyl-4-propylamino-4H-thieno-[2,3-b]thiopyran-2-sulfonamide 7,7-dioxide.

3. The composition of claim 2, wherein the carbonic anhydrase inhibitor is selected from the group consisting of: (R)-3,4-Dihydro-4-ethylamine-2-(3-methoxy)propyl-2H-thieno[3,2-e]-1,2-thiazine-6-sulfonamide 1,1-dioxide and (R)-3,4-Dihydro-4-ethylamino-2-(2-methoxy)ethyl-2H-thieno[3,2-e]-1,2-thiazine-6-sulfonamide 1,1-dioxide; (R)-4-ethylamino-3,4-dihydro-2-(4-methoxy)butyl-2H-thieno[3,2-e]-1,2-thiazine-6-sulfonamide 1,1-dioxide; (R)-3,4-dihydro-2-(4-methoxy)butyl-4-propylamino-2-thieno[3,2-e]-1,2-thiazine-6-sulfonamide 1,1-dioxide; (-)-trans-5,6-dihydro-6-(3-methoxy)propyl-4-propylamino-4H-thieno-[2,3-b]thiopyran-2-sulfonamide 7,7-dioxide.

4. The composition of claim 1, wherein the carbonic anhydrase inhibitor is selected from the group consisting of: (R)-3,4-Dihydro-2-(2-ethoxy)ethyl-4-ethylamino-2H-thieno[3,2-e]-1,2-thiazine-6-sulfonamide 1,1-dioxide hydrochloride; (R)-3,4-Dihydro-2-(2-ethoxy)ethyl-4-propylamino-2H-thieno[3,2-e]-1,2-thiazine-6-sulfonamide 1,1-dioxide hydrochloride; (R)-3,4-Dihydro-4-ethylamino-2-(3-methoxy)propyl-2H-thieno[3,2-e]-1,2-thiazine-6-sulfonamide 1,1-dioxide hydrochloride; (R)-3,4-Dihydro-2-(3-methoxy)propyl-4-propylamino-2H-thieno[3,2-e]-1,2-thiazine-6-sulfonamide 1,1-dioxide hydrochloride; (R)-3,4-Dihydro-4-ethylamino-2-[2-methoxyethoxy)ethyl]-2H-thieno[3,2-e]-1,2-thiazine-6-sulfonamide 1,1-dioxide hydrochloride: (R)-3,4-Dihydro-2-[2-methoxyethoxy)ethyl]-4-propylamino-2H-thieno[3,2-e]-1,2-thiazine-6-sulfonamide 1,1-dioxide hydrochloride: (R)-3,4-Dihydro-4-ethylamino-2-[3-(2-methoxy)ethoxy]propyl-2H-thieno[3,2-e]-1,2-thiazine-6-sulfonamide 1,1-dioxide hydrochloride; (R)-3,4-Dihydro-2-[3-(methoxyethoxy)propyl]-4-propylamino-2H-thieno[3,2-e]-1,2-thiazine-6-sulfonamide 1,1-dioxide hydrochloride; (R)-3,4-Dihydro-4-ethylamino-2-(2-methoxy)ethyl-2H-thieno[3,2-e]-1,2-thiazine-6-sulfonamide 1,1-dioxide hydrochloride; (R)-3,4-Dihydro-2-(2-methoxy)ethyl-4-propylamino--2H-thieno[3,2-e]-1,2-thiazine-6-sulfonamide 1,1-dioxide hydrochloride; (R)-3,4-Dihydro-4-ethylamino-2-methyl-2H-thieno[3,2-e]-1,2-thiazine-6-sulfonamide 1,1-dioxide hydrochloride; (R)-4-ethylamino-3,4-dihydro-2-(4-methoxy)butyl-2H-thieno[3,2-e]-1,2-thiazine-6-sulfonamide 1,1-dioxide; (R)-3,4-dihydro-2-(4-methoxy)butyl-4-propylamino-2-thieno[3,2-e]-1,2-thiazine-6-sulfonamide 1,1-dioxide; (R)-4-Ethylamino-2-(4-methoxyphenyl)-3,4-dihydro-2H-thieno[3,2-e]-1,2-thiazine-6-sulfonamide 1,1-dioxide hydrochloride; (R)-4-Ethylamino-3,4-dihydro-2-(3-methoxyphenyl)-2H-thieno[3,2-e]-1,2-thiazine-6-sulfonamide 1,1-dioxide hydrochloride; (R)-4-Ethylamino-2-(4-hydroxyphenyl)-3,4-dihydro-2H-thieno[3,2-e]-1,2-thiazine-6-sulfonamide 1,1-dioxide hydrochloride; (R)-4-Ethylamino-3,4-dihydro-2-(3-hydroxyphenyl)-2H-thieno[3,2-e]-1,2-thiazine-6-sulfonamide 1,1-dioxide hydrochloride; (R)-4-Ethylamino-3,4-dihydro-2-(3-hydroxyphenylmethyl)-2H-thieno[3,2-e]-1,2 thiazine-6-sulfonamide 1,1-dioxide hydrochloride; (R)-4-Ethylamino-3,4-dihydro-2-(3-methoxyphenylmethyl)-2H-thieno[3,2-e]-1,2-thiazine-6-sulfonamide 1,1-dioxide hydrochloride; (R)-4-Ethylamino-3,4-dihydro-2-(2-methylpropyl)-2H-thieno[3,2-e]-1,2-thiazine-6-sulfonamide 1,1-dioxide hydrochloride; (R)-4-Ethylamino-3,4-dihydro-2-(6-hydroxyhexyl)-2H-thieno[3,2-e]-1,2-thiazine-6-sulfonamide 1,1-dioxide hydrochloride; (R)-3,4-Dihydro-2-(3-hydroxypropyl)-4-(2-methylpropyl)amino-2H-thieno[3,2-e]-1,2-thiazine-6-sulfonamide 1,1-dioxide hydrochloride hemihydrate; and (-)-trans-5,6-dihydro-6-(3-methoxy)propyl-4-propylamino-4H-thieno-[2,3-b]thiopyran-2-sulfonamide 7,7-dioxide.

5. The composition of claim 4, wherein the carbonic anhydrase inhibitor is selected from the group consisting of: (R)-3,4-Dihydro-4-ethylamino-2-(3-methoxy)propyl-2H-thieno[3,2-e]-1,2-thiazine-6-sulfonamide 1,1-dioxide hydrochloride and (R)-3,4-Dihydro-4-ethylamino-2-(2-methoxy)ethyl-2H-thieno[3,2-e]-1,2-thiazine-6-sulfonamide 1,1-dioxide hydrochloride; (R)-4-ethylamino-3,4-dihydro-2-(4-methoxy)butyl-2H-thieno[3,2-e]-1,2-thiazine-6-sulfonamide 1,1-dioxide; (R)-3,4-dihydro-2(4-methoxy)butyl-4-propylamino-2-thieno[3,2-e]-1,2-thiazine-6-sulfonamide 1,1-dioxide; (-)-trans-5,6-dihydro-6-(3-methoxy)propyl-4-propylamino-4H-thieno-[2,3-b]thiopyran-2-sulfonamide 7,7-dioxide.

6. The composition of any of claims 1 to 5, wherein the final composition concentration of beta-blocker is less than or equal to about 2.0 wt%, and the final composition concentration of carbonic anhydrase inhibitor is less than or equal to about 5 wt%.

7. The composition of claim 6, wherein the final composition concentration of the beta-blocker is between about 0.1 and about 1.0 wt%.

8. The composition of claim 7, wherein the final composition concentration of the beta-blocker is between about 0.25 and about 0.5 wt%.

9. The composition of claim 8 wherein the final composition concentration of the beta-blocker is 0.25 wt%.

10. The composition of claim 6, wherein the final composition concentration of the carbonic anhydrase inhibitor is between about 0.25 and about 3 wt%.

11. The composition of claim 10, wherein the final composition concentration of the carbonic anhydrase inhibitor is between about 0.5 and about 2 wt.%.

12. The composition of any of claims 1 to 11, wherein the beta-blocker is selected from the racemic and enantiomeric forms of: betaxolol, timolol, metoprolol, befunolol, falintolol, levobunolol, carteolol, mepindolol, pindolol, bisoprolol, bopindolol, atenolol, arotinolol, acebutolol, nadolol, celiprolol, metipranolol, bevantolol, ICI 118,551, pamatolol, penbutolol, toliprolol, tiprenolol, practolol, procinolol, exaprolol, cicloprolol, carazolol, tazolol, tienoxolol, oxprenolol, propranolol, IPS 339, labetolol, dilevalol, esmolol, bupranolol, bunolol, isoxaprolol, diacetolol, hydroxylevobunolol, carvedilol, and their pharmaceutically acceptable salts.

13. The composition of claim 12, wherein the beta-blocker is selected from the racemic and enantiomeric forms of: betaxolol, timolol, carteolol, levobunolol and hydroxylevobunolol, and their pharmaceutically acceptable salts.

14. The composition of claim 13, wherein the beta-blocker is betaxolol or a pharmaceutically acceptable salt thereof.

15. The composition of claim 13, wherein the beta-blocker is S-timolol or a pharmaceutically acceptable salt thereof.

16. The composition of any of claims 1 to 11, wherein the beta-blocker is a thiadiazole of formula: 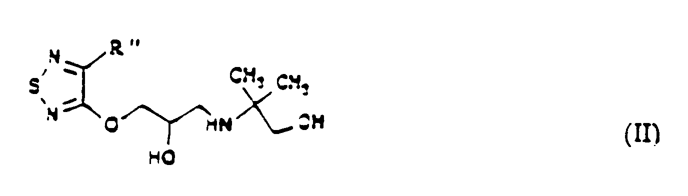 and optically active isomers and pharmacologically acceptable salts thereof, wherein R'' is selected from the group consisting of: hydrogen, halogen, C₁₋₅ alkyl, C₂₋₅ mono-alkenyl, C₂₋₅ alkoxy, C₃₋₆ cycloalkyl, phenyl, phenalkyl, morpholino, furyl, thienyl and pyrryl.

17. The composition of c!aim 16, wherein R'' is selected from the group consisting of: chlorine, ethyl, allyl, cyclopropyl, ethoxy, phenyl, phenyl-chloromethyl and 2-(cyclopropylmethoxy)ethyl.

18. The composition of any of claims 1 to 17, further comprising an anionic mucomimetic polymer wherein the final composition concentration of the anionic mucomimetic polymer is between about 0.05 and about 8.0 wt%.

19. The composition of claim 18 ,further comprising a finely-divided drug carrier substrate, wherein the final composition concentration of the finely-divided drug carrier substrate is between about 0.05 and about 10.0 wt%.

20. A composition according to any of claims 1 to 19 for use in the treatment of glaucoma and ocular hypertension.

21. Use of a composition comprising a beta-blocker and a carbonic anhydrase inhibitor in an ophthalmically acceptable vehicle, wherein: the final composition concentration of the beta-blocker is less than or equal to about 2.0 wt%, and the final composition concentration of the carbonic anhydrase inhibitor is less than or equal to about 5 wt% and the carbonic anhydrase inhibitor has the formula: 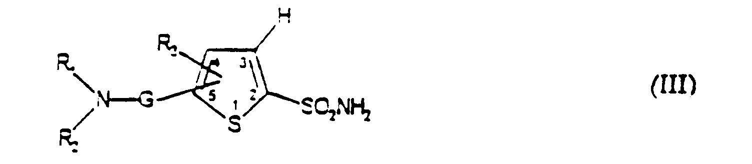 or a pharmaceutically acceptable salt thereof, wherein:
R₁ is: H; C₁₋₄ alkyl; C₂₋₄ alkyl substituted optionally with OH, halogen, C₁₋₄ alkoxy or C(=O)R₇;
R₂ is: H; C₁₋₈ alkyl; C₂₋₈ alkyl substituted with OH, NR₅R₆, halogen, C₁₋₄ alkoxy, C₂₋₄alkoxyC₁₋₄alkoxy, OC(=O)R₇, or C(=O)R₇; C₃₋₇ alkenyl unsubstituted or substituted optionally with OH, NR₅R₆, or C₁₋₄ alkoxy; C₃₋₇alkynyl unsubstituted or substituted optionally with OH, NR₅R₆, or C₁₋₄ alkoxy; C₁₋₃ alkyl substituted with phenyl or R₁₀ either of which can be unsubstituted or substituted optionally with C₁₋₃ alkyl, C₁₋₃ haloalkyl, OH, (CH₂)ₙNR₅R₆, halogen, C₁₋₄ alkoxy, C₁₋₄ haloalkoxy, C(=O)R₇, S(=O)ₘR₈ or SO₂NR₅R₆, wherein m is 0 - 2 and n is 0 - 2; C₂₋₄ alkoxy substituted optionally with NR₅R₆, halogen, C₁₋₄ alkoxy, or C(=O)R₇; phenyl or R₁₀ either of which can be unsubstituted or substituted optionally with C₁₋₃ alkyl, C₁₋₃ haloalkyl, OH, (CH₂)ₙNR₅R₆, halogen, C₁₋₄ alkoxy, C₁₋₄ haloalkoxy, C(=O)R₇, S(=O)ₘR₈ or SO₂NR₅R₆, wherein m is 0 - 2 and n is 0 - 2; provided that R₁ and R₂ cannot both be H; or R₁ and R₂ can be joined to form a saturated ring of 5 or 6 atoms selected from O, S, C or N, such as, pyrrolidine, oxazolidine, thiomorpholine, thiomorpholine 1,1 dioxide, morpholine, piperazine, thiazolidine 1,1 dioxide, or tetrahydrooxazine, which can be unsubstituted or substituted optionally on carbon with OH, NR₅R₆, halogen, C₁₋₄ alkoxy, C(=O)R₇, C₁₋₆ alkyl, C₁₋₆ alkyl substituted optionally with OH, NR₅R₆, halogen, C₁₋₄ alkoxy, C(=O)R₇ or on nitrogen with NR₅R₆, C₁₋₄ alkoxy, C(=O)R₇, C₁₋₆ alkyl or C₂₋₆ alkyl substituted optionally with OH, NR₅R₆, halogen, C₁₋₄ alkoxy or C=(=O)R₇;
R₃ is: H; halogen; C₁₋₄ alkyl; C₁₋₈ alkoxy; C₁₋₈ alkylthiol; C₂₋₈ alkoxy substituted optionally with OH, NR₅R₆, halogen, C₁₋₄ alkoxy or C(=O) R₇; C₁₋₄ alkyl substituted optionally with R₄; or R₁ and R₃ can be joined together with carbon atoms to form a ring of from 5 to 7 members in which said carbon atoms can be unsubstituted or substituted optionally with R₄;
R₄ is: OH; C₁₋₄ alkyl unsubstituted or substituted optionally with OH, NR₅R₆, halogen, C₁₋₄ alkoxy or C(=O)R₇; C₁₋₄ alkoxy; C₂₋₄ alkoxy substituted optionally with OH, NR₅R₆, halogen, C₁₋₄ alkoxy or C(=O)R₇; NR₅R₆; phenyl or R₁₀ either of which can be unsubstituted or substituted optionally with OH, (CH₂)ₙNR₅R₆, halogen, C₁₋₄ alkoxy, C₁₋₄ haloalkoxy, C(=O)R₇, S(=O)ₘR₈ or SO₂NR₅R₆, wherein m is 0 - 2 and n is 0 - 2;
Provided that when G is SO₂ and R₃ is in the 4 position and is H or halogen then R₁ and R₂ are not H, C₁₋₆ alkyl substituted optionally with OH, C₁₋₆ alkoxy, C₂₋₆ alkoxycarbonyl, C₂₋₆ alkenyl, phenyl, phenoxy, pyridyl, tetrahydrofuryl, C₂₋₆ alkanoyl, C₂₋₆ alkenyl, nor are they joined to form a 5, 6 or 7 member ring, saturated or unsaturated, comprised of atoms selected optionally from C, O, S, N in which said nitrogen, when saturated, is substituted optionally with H or C₁₋₆ alkyl or in which said carbon is substituted optionally with C₁₋₆ alkyl, C₁₋₆ alkoxy or OH; and when R₃ is in the 5 position and is H, Cl, Br, or C₁₋₃ alkyl, then neither R₁ nor R₂ can be H or C₁₋₄ alkyl; and when G is C(=O) and in the 5- position and R₃ is H, then R₁ and R₂ cannot both be CH₃;
R₅ & R₆ are the same or different and are: H; C₁₋₄ alkyl; C₂₋₄ alkyl substituted optionally with OH, halogen, C₁₋₄ alkoxy or C(=O)R₇; C₁₋₄ alkoxy; C₂₋₄ alkoxy substituted optionally with OH, halogen, C₁₋₄ alkoxy or C(=O)R₇; C₃₋₇ alkenyl unsubstituted or substituted optionally with OH, NR₅R₆, or C₁₋₄ alkoxy; C₃₋₇ alkynyl unsubstituted or substituted optionally with OH, NR₅R₆, or C₁₋₄ alkoxy; C₁₋₂alkyl-C₃₋₅cycloalkyl; C(=O)R₇ or R₅ and R₆ can be joined to form a ring of 5 or 6 atoms selected from O, S, C or N, such as, pyrrolidine, oxazolidine, thiomorpholine, thiomorpholine 1.1 dioxide, morpholine, piperazine or thiazolidine 1,1-dioxide, which can be unsubstituted or substituted optionally on carbon with OH, (=O), halogen, C₁₋₄ alkoxy, C(=O)R₇, C₁₋₆ alkyl, C₁₋₆ alkyl substituted optionally with OH, halogen, C₁₋₄ alkoxy, C(=O)R₇ or on nitrogen with C₁₋₄ alkoxy, C(=O)R₇, S(=O)ₘR₈, C₁₋₆ alkyl or C₂₋₆ alkyl substituted optionally with OH, halogen, C₁₋₄ alkoxy, C(=O)R₇ or on sulfur by (=O)ₘ, wherein m is 0 - 2;
R₇ is: C₁₋₈ alkyl; C₁₋₈ alkyl, substituted optionally with OH, NR₅R₆, halogen, C₁₋₄ alkoxy or C(=O)R₉; C₁₋₄ alkoxy; C₂₋₄ alkoxy substituted optionally with OH, NR₅R₆, halogen or C₁₋₄ alkoxy; NR₅R₆; or phenyl or R₁₀ either of which can be unsubstituted or substituted optionally with OH, halogen, C₁₋₃ alkyl, C₁₋₃ haloalkoxy, (CH₂)ₙNR₅R₆, S(=O)ₘR₈ or SO₂NR₅R₆, wherein n is 0 or 1 and m is 0-2;
R₈ is: C₁₋₄ alkyl; C₂₋₄ alkyl substituted optionally with OH, NR₅R₆, halogen, C₁₋₄ alkoxy or C(=O)R₇;
R₉ is: C₁₋₄ alkyl; C₁₋₄ alkoxy; amino, C₁₋₃ alkylamino, or di-C₁₋₃ alkylamino;
R₁₀ is: a monocyclic ring system of 5 or 6 atoms composed of C, N, O, and/or S, such as furan, thiophene, pyrrole, pyrazole, imidazole, triazole, tetrazole, oxazole, isoxazole, isothiazole, thiazole, thiadiazole, pyridine, pyrimidine, pyridazine, and pyrazine; and
G is: C(=O) or SO₂ or
wherein the carbonic anhydrase inhibitor is (-)-trans-5,6-dihydro-6-(3-methoxy)propyl-4-propylamino-4H-thieno[2,3-b]thiopyran-2-sulfonamide 7,7-dioxide for the preparation of a medicament for the treatment of glaucoma and ocular hypertension.

22. Use of claim 21, wherein the carbonic anhydrase inhibitor is selected from the group consisting of: (R)-3,4-Dihydro-2-(2-ethoxy)ethyl-4-ethylamino-2H-thieno[3,2-e]-1,2-thiazine-6-sulfonamide 1,1-dioxide; (R)-3,4-Dihydro-2-(2-ethoxy)ethyl-4-propylamino-2H-thieno[3,2-e]-1,2-thiazine-6-sulfonamide 1,1-dioxide; (R)-3,4-Dihydro-4-ethylamino-2-(3-methoxy)propyl-2H-thieno[3,2-e]-1,2-thiazine-6-sulfonamide 1,1-dioxide; (R)-3,4-Dihydro-2-(3-methoxy)propyl-4-propylamino-2H-thieno[3,2-e]-1,2-thiazine-6-sulfonamide 1,1-dioxide; (R)-3,4-Dihydro-4-ethylamino-2-[2-methoxyethoxy)ethyl]-2H-thieno[3,2-e]-1,2-thiazine-6-sulfonamide 1,1-dioxide; (R)-3,4-Dihydro-2-[2-methoxyethoxy)ethyl]-4-propylamino-2H-thieno[3,2-e]-1,2-thiazine-6-sulfonamide 1,1-dioxide; (R)-3,4-Dihydro-4-ethylamino-2-[3-(2-methoxy)ethoxy]propyl-2H-thieno[3,2-e]-1,2-thiazine-6-sulfonamide 1,1-dioxide; (R)-3,4-Dihydro-2-[3-(methoxyethoxy)propyl]-4-propylamino-2H-thieno[3,2-e]-1,2-thiazine-6-sulfonamide 1,1-dioxide; (R)-3,4-Dihydro-4-ethylamino-2-(2-methoxy)ethyl-2H-thieno[3,2-e]-1,2-thiazine-6-sulfonamide 1,1-dioxide; (R)-3,4-Dihydro-2-(2-methoxy)ethyl-4-propylamino-2H-thieno[3,2-e]-1,2-thiazine-6-sulfonamide 1,1-dioxide; (R)-3,4-Dihydro-4-ethylamino-2-methyl-2H-thieno[3,2-e]-1,2-thiazine-6-sulfonamide 1,1-dioxide; (R)-4-ethylamino-3,4-dihydro-2-(4-methoxy)butyl-2H-thieno[3,2-e]-1,2-thiazine-6-sulfonamide 1,1-dioxide; (R)-3,4-dihydro-2-(4-methoxy)butyl-4-propylamino-2-thieno[3,2-e]-1,2-thiazine-6-sulfonamide 1,1-dioxide; (R)-4-Ethylamino-2-(4-methoxyphenyl)-3,4-dihydro-2H-thieno[3,2-e]-1,2-thiazine-6-sulfonamide 1,1-dioxide; (R)-4-Ethylamino-3,4-dihydro-2-(3-methoxyphenyl)-2H-thieno[3,2-e]-1,2-thiazine-6-sulfonamide 1,1-dioixide; (R)-4-Ethylamino-2-(4-hydroxyphenyl)-3,4-dihydro-2H-thieno[3,2-e]-1,2-thiazine-6-sulfonamide 1,1-dioxide; (R)-4-Ethylamino-3,4-dihydro-2-(3-hydroxyphenyl)-2H-thieno[3,2-e]-1,2-thiazine-6-sulfonamide 1,1-dioxide; (R)-4-Ethylamino-3,4-dihydro-2-(3-hydroxyphenylmethyl)-2H-thieno[3,2-e]-1,2-thiazine-6-sulfonamide 1,1-dioxide; (R)-4-Ethylamino-3,4-dihydro-2-(3-methoxyphenylmethyl)-2H-thieno[3,2-e]-1,2-thiazine-6-sulfonamide 1,1-dioxide; (R)-4-Ethylamino-3,4-dihydro-2-(2-methylpropyl)-2H-thieno[3,2-e]-1,2-thiazine-6-sulfonamide 1,1-dioxide; (R)-4-Ethylamino-3,4-dihydro-2-(6-hydroxyhexyl)-2H-thieno[3,2-e]-1,2-thiazine-6-sulfonamide 1,1-dioxide; (R)-3,4-Dihydro-2-(3-hydroxypropyl)-4-(2-methylpropyl)amino-2H-thieno[3,2-e]-1,2-thiazine-6-sulfonamide 1,1-dioxide; and (-)-trans-5,6-dihydro-6-(3-methoxy)propyl-4-propylamino-4H-thieno-[2,3-b]thiopyran-2-sulfonamide 7,7-dioxide.

23. Use of claim 21, wherein the carbonic anhydrase inhibitor is selected from the group consisting of: (R)-3,4-Dihydro-4-ethylamino-2-(3-methoxy)propyl-2H-thieno[3,2-e]-1,2-thiazine-6-sulfonamide 1,1-dioxide and (R)-3,4-Dihydro-4-ethylamino-2-(2-methoxy)ethyl-2H-thieno[3,2-e]-1,2-thiazine-6-sulfonamide 1,1-dioxide; (R)-4-ethylamino-3,4-dihydro-2-(4-methoxy)butyl-2H-thieno[3,2-e]-1,2-thiazine-6-sulfonamide 1,1-dioxide; (R)-3,4-dihydro-2-(4-methoxy)butyl-4-propylamino-2-thieno[3,2-e]-1,2-thiazine-6-sulfonamide 1,1-dioxide; (-)-trans-5,6-dihydro-6-(3-methoxy)propyl-4-propylamino-4H-thieno-[2,3-b]thiopyran-2-sulfonamide 7,7-dioxide.

24. Use of claim 21, wherein the carbonic anhydrase inhibitor is selected from the group consisting of: (R)-3,4-Dihydro-2-(2-ethoxy)ethyl-4-ethylamino-2H-thieno[3,2-e]-1,2-thiazine-6-sulfonamide 1,1-dioxide hydrochloride; (R)-3,4-Dihydro-2-(2-ethoxy)ethyl-4-propylamino-2H-thieno[3,2-e]-1,2-thiazine-6-sulfonamide 1,1-dioxide hydrochloride; (R)-3,4-Dihydro-4-ethylamino-2- ( 3-methoxy) propyl-2H-thieno[3,2-e]-1,2-thiazine-6-sulfonamide 1,1-dioxide hydrochloride; (R)-3,4-Dihydro-2-(3-methoxy)propyl-4-propylamino-2H-thieno[3,2-e]-1,2-thiazine-6-sulfonamide 1,1-dioxide hydrochloride; (R)-3,4-Dihydro-4-ethylamino-2-[2-methoxyethoxy)ethyl]-2H-thieno[3,2-e]-1,2-thiazine-6-sulfonamide 1,1-dioxide hydrochloride; (R)-3,4-Dihydro-2-[2-methoxyethoxy)ethyl]-4-propylamino-2H-thieno[3,2-e]-1,2-thiazine-6-sulfonamide 1,1-dioxide hydrochloride; (R)-3,4-Dihydro-4-ethylamino-2-[3-(2-methoxy)ethoxy]propyl-2H-thieno[3,2-e]-1,2-thiazine-6-sulfonamide 1,1-dioxide hydrochloride; (R)-3,4-Dihydro-2-[3-(methoxyethoxy)propyl]-4-propylamino-2H-thieno[3,2-e]-1,2-thiazine-6-sulfonamide 1,1-dioxide hydrochloride; (R)-3,4-Dihydro-4-ethylamino-2-(2-methoxy)ethyl-2H-thieno[3,2-e]-1,2-thiazine-6-sulfonamide 1,1-dioxide hydrochloride; (R)-3,4-Dihydro-2-(2-methoxy)ethyl-4-propylamino-2H-thieno[3,2-e]-1,2-thiazine-6-sulfonamide 1,1-dioxide hydrochloride; (R)-3,4-Dihydro-4-ethylamino-2-methyl-2H-thieno[3,2-e]-1,2-thiazine-6-sulfonamide 1,1-dioxide hydrochloride; (R)-4-ethylamino-3,4-dihydro-2-(4-methoxy)butyl-2H-thieno[3,2-e]-1,2-thiazine-6-sulfonamide 1,1-dioxide; (R)-3,4-dihydro-2-(4-methoxy)butyl-4-propylamino-2-thieno[3,2-e]-1,2-thiazine-6-sulfonamide 1,1-dioxide; (R)-4-Ethylamino-2-(4-methoxyphenyl)-3,4-dihydro-2H-thieno[3,2-e]-1,2-thiazine-6-sulfonamide 1,1-dioxide hydrochloride; (R)-4-Ethylamino-3,4-dihydro-2-(3-methoxyphenyl)-2H-thieno[3,2-e]-1,2-thiazine-6-sulfonamide 1,1-dioxide hydrochloride; (R)-4-Ethylamino-2-(4-hydroxyphenyl)-3,4-dihydro-2H-thieno[3,2-e]-1,2-thiazine-6-sulfonamide 1,1-dioxide hydrochloride; (R)-4-Ethylamino-3,4-dihydro-2-(3-hydroxyphenyl)-2H-thieno[3,2-e]-1,2-thiazine-6-sulfonamide 1,1-dioxide hydrochloride; (R)-4-Ethylamino-3,4-dihydro-2-(3-hydroxyphenylmethyl)-2H-thieno[3,2-e]-1,2-thiazine-6-sulfonamide 1,1-dioxide hydrochloride; (R)-4-Ethylamino-3,4-dihydro-2-(3-methoxyphenylmethyl)-2H-thieno[3,2-e]-1,2-thiazine-6-sulfonamide 1,1-dioxide hydrochloride; (R)-4-Ethylamino-3,4-dihydro-2-(2-methylpropyl)-2H-thieno[3,2-e]-1,2-thiazine-6-sulfonamide 1,1-dioxide hydrochloride; (R)-4-Ethylamino-3,4-dihydro-2-(6-hydroxyhexyl)-2H-thieno[3,2-e]-1,2-thiazine-6-sulfonamide 1,1-dioxide hydrochloride; (R)-3,4-Dihydro-2-(3-hydroxypropyl)-4-(2-methylpropyl)amino-2H-thieno[3,2-e]-1,2-thiazine-6-sulfonamide 1,1-dioxide hydrochloride hemihydrate; and (-)-trans-5,6-dihydro-6-(3-methoxy)propyl-4-propylamino-4H-thieno-[2,3-b]thiopyran-2-sulfonamide 7,7-dioxide.

25. Use of claim 21, wherein the carbonic anhydrase inhibitor is selected from the group consisting of: (R)-3,4-Dihydro-4-ethylamino-2-(3-methoxy)propyl-2H-thieno[3,2-e]-1,2-thiazine-6-sulfonamide 1,1-dioxide hydrochloride and (R)-3,4-Dihydro-4-ethylamino-2-(2-methoxy)ethyl-2H-thieno[3,2-e]-1,2-thiazine-6-sulfonamide 1,1-dioxide hydrochloride; (R)-4-ethylamino-3,4-dihydro-2-(4-methoxy)butyl-2H-thieno[3,2-e]-1,2-thiazine-6-sulfonamide 1,1-dioxide; (R)-3,4-dihydro-2-(4-methoxy)butyl-4-propylamino-2-thieno[3,2-e]-1,2-thiazine-6-sulfonamide 1,1-dioxide: (-)-trans-5,6-dihydro-6-(3-methoxy)propyl-4-propylamino-4H-thieno-[2,3-b]thiopyran-2-sulfonamide 7,7-dioxide.

26. Use of any of claims 21 to 25, wherein the beta-blocker is selected from the racemic and enantiomeric forms of: betaxolol, timolol, metoprolol, befunolol, falintolol, levobunolol, carteolol, mepindolol, pindolol, bisoprolol, bopindolol, atenolol, arotinolol, acebutolol, nadolol, celiprolol, metipranolol, bevantolol, ICI 118,551, pamatolol, penbutolol, toliprolol, tiprenolol, practolol, procinolol, exaprolol, cicloprolol, carazolol, tazolol, tienoxolool, oxprenolol, propranolol, IPS 339, labetolol, dilevalol, esmolol, bupranolol, bunolol, isoxaprolol, diacetolol and hydroxylevobunolol, and their pharmaceutically acceptable salts.

27. Use of claim 26, wherein the beta-blocker is selected from the group consisting of: betaxolol, timolol, carteolol, levobunolol and hydroxylevobunolol, and their pharmaceutically acceptable salts.

28. Use of any of claims 21 to 27, wherein the beta-blocker is a thiadiazole of formula: 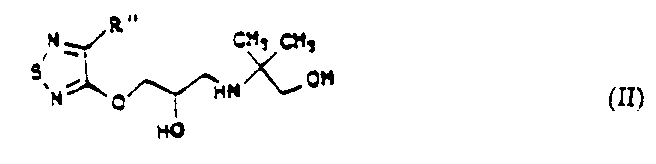 and optically active isomers and pharmacologically acceptable salts thereof, wherein R'' is selected from the group consisting of: hydrogen, halogen, C₁₋₅ alkyl, C₂₋₅ mono-alkenyl, C₂₋₅ alkoxy, C₃₋₆ cycloalkyl, phenyl, phenalkyl, morpholino, furyl, thienyl and pyrryl.

29. Use of claim 28, wherein R'' is selected from the group consisting of: chlorine, ethyl, allyl, cyclopropyl, ethoxy, phenyl, phenyl-chloromethyl and 2- (cyclopropylmethoxy)ethyl.

## Patentansprüche

1. Topische ophthalmische Zusammensetzung umfassend einen Betablocker und einen Carboanhydrasehemmer in einem ophthalmisch annehmbaren Träger, wobei die Zusammensetzung eine Suspension ist und die endgültige Zusammensetzung einen pH von etwa 5,0 bis etwa 7,8 hat und der Carboanhydrasehemmer die Formel: 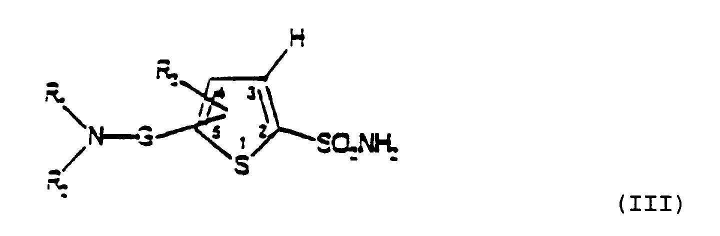 hat, oder ein pharmazeutisch annehmbares Salz davon, worin
R₁ H, ein C₁-C₄-Alkylrest, C₂-C₄-Alkylrest, der gegebenenfalls mit OH, Halogen, C₁-C₄-Alkoxyresten oder C(=O)R₇ substituiert ist,
R₂ H, ein C₁-C₈-Alkylrest, C₂-C₈-Alkylrest, der mit OH, NR₅R₆, Halogen, C₁-C₄-Alkoxyresten, C₂-C₄-Alkoxy-C₁-C₄-alkoxyresten, OC(=O)R₇ oder C(=O)R₇ substituiert ist, C₃-C₇-Alkenylrest, der unsubstituiert oder gegebenenfalls mit OH, NR₅R₆ oder C₁-C₄-Alkoxyresten substituiert ist, C₃-C₇-Alkinylrest, der unsubstituiert oder gegebenenfalls mit OH, NR₅R₆ oder C₁-C₄-Alkoxyresten substituiert ist, C₁-C₃-Alkylrest, der mit Phenylresten oder R₁₀ substituiert ist, die jeweils unsubstituiert oder gegebenenfalls mit C₁-C₃-Alkylresten, C₁-C₃-Halogenalkylresten, OH, (CH₂)ₙNR₅R₆, Halogen, C₁-C₄-Alkoxyresten, C₁-C₄-Halogenalkoxyresten, C(=O)R₇, S(=O)ₘR₈ oder SO₂NR₅R₆ substituiert sein können, wobei m 0 bis 2 ist und n 0 bis 2 ist, C₂-C₄-Alkoxyrest, der gegebenenfalls mit NR₅R₆, Halogen, C₁-C₄-Alkoxyresten oder C(=O)R₄ substituiert ist, ein Phenylrest oder R₁₀ ist, die jeweils unsubstituiert oder gegebenenfalls mit C₁-C₃-Alkylresten, C₁-C₃-Halogenalkylresten, OH, (CH₂)ₙNR₅R₆, Halogen, C₁-C₄-Alkoxyresten, C₁-C₄-Halogenalkoxyresten, C(=O)R₇, S(=O)ₘR₈ oder SO₂NR₅R₆ substituiert sein können, worin m 0 bis 2 ist und n 0 bis 2 ist, mit dem Vorbehalt, daß R₁ und R₂ nicht beide H sein können, oder R₁ und R₂ verbunden sein können unter Bildung eines gesättigten Rings mit 5 oder 6 Atomen ausgewählt aus O, S, C oder N, z.B. eines Pyrrolidin-, Oxazolidin-, Thiomorpholin-, Thiomorpholin-1,1-dioxid-, Morpholin-, Piperazin-, Thiazolidin-1,1-dioxid- oder Tetrahydrooxazinrings, der unsubstituiert oder gegebenenfalls am Kohlenstoff mit OH, NR₅R₆, Halogen, C₁-C₄-Alkoxyresten, C(=O)R₇, C₁-C₆-Alkylresten, C₁-C₆-Alkylresten, die gegebenenfalls mit OH, NR₅R₆, Halogen-, C₁-C₄-Alkoxyresten, C(=O)R₇ oder am Stickstoff mit NR₅R₆, C₁-C₄-Alkoxyresten, C(=O)R₇, C₁-C₆-Alkylresten oder C₂-C₆-Alkylresten, die gegebenenfalls mit OH, NR₅R₆, Halogen, C₁-C₄-Alkoxyresten oder C(=O)R₇ substituiert sind, substituiert sein kann,
R₃ H, Halogen, ein C₁-C₄-Alkylrest, C₁-C₈-Alkoxyrest, C₁-C₈-Alkylthiolrest, C₂-C₈-Alkoxyrest, der gegebenenfalls mit OH, NR₅R₆, Halogen, C₁-C₄-Alkoxyresten oder C(=O)R₇ substituiert ist, ein gegebenenfalls mit R₄ substituierter C₁-C₄-Alkylrest ist, oder worin R₁ und R₃ miteinander verbunden sein können zusammen mit Kohlenstoffatomen unter Bildung eines Rings mit 5 bis 7 Gliedern, wobei die Kohlenstoffatome unsubstituiert oder gegebenenfalls mit R₄ substituiert sein können,
R₄ OH, ein unsubstituierter oder gegebenenfalls mit OH, NR₅R₆, Halogen, C₁-C₄-Alkoxyresten oder C(=O)R₇ substituierter C₁-C₄-Alkylrest, C₁-C₄-Alkoxyrest, gegebenenfalls mit OH, NR₅R₆, Halogen, C₁-C₄-Alkoxyresten oder C(=O)R₇ substituierter C₂-C₄-Alkoxyrest, NR₅R₆, Phenylrest oder R₁₀ ist, von denen jeder unsubstituiert oder gegebenenfalls mit OH, (CH₂)ₙNR₅R₆, Halogen, C₁-C₄-Alkoxyresten, C₁-C₄-Halogenalkoxyresten, C(=O)R₇, S(=O)ₘR₈ oder SO₂NR₅R₆ substituiert sein kann, worin m 0 bis 2 ist und n 0 bis 2 ist,
mit dem Vorbehalt, daß dann, wenn G SO₂ ist und R₃ in Position 4 ist und H oder Halogen ist, daß dann R₁ und R₂ nicht H, gegebenenfalls mit OH substituierte C₁-C₆-Alkylreste, C₁-C₆-Alkoxyreste, C₂-C₆-Alkoxycarbonylreste, C₂-C₆-Alkenylreste, Phenyl-, Phenoxy-, Pyridyl-, Tetrahydrofuryl-, C₂-C₆-Alkanoyl-, C₂-C₆-Alkenylreste sind, noch unter Bildung eines 5-, 6- oder 7-gliedrigen Rings verbunden sind, der gesättigt oder ungesättigt sein kann und aus Atomen gegebenenfalls ausgewählt aus C, O, S und N aufgebaut ist, wobei der Stickstoff, wenn er gesättigt ist, gegebenenfalls mit H oder C₁-C₆-Alkylresten substituiert ist, oder worin der Kohlenstoff gegebenenfalls mit C₁-C₆-Alkyl-, C₁-C₆-Alkoxyresten oder OH substituiert ist und wenn R₃ in Position 5 ist und H, Cl, Br oder ein C₁-C₃-Alkylrest ist, daß dann weder R₁ noch R₂ H oder ein C₁-C₄-Alkylrest sein können und wenn G C(=O) ist und in Position 5 ist und R₃ H ist, daß dann R₁ und R₂ nicht beide CH₃ sein können;
R₅ und R₆ gleich oder verschieden sind und H, C₁-C₄-Alkylreste, gegebenenfalls mit OH, Halogen, C₁-C₄-Alkoxyresten oder C(=O)R₇ substituierte C₂-C₄-Alkylreste, C₁-C₄-Alkoxyreste, gegebenenfalls mit OH, Halogen, C₁-C₄-Alkoxyresten oder C(=O)R₇ substituierte C₂-C₄-Alkoxyreste, C₃-C₇-Alkenylreste, die unsubstituiert oder gegebenenfalls mit OH, NR₅R₆ oder C₁-C₄-Alkoxyresten substituiert sind, C₃-C₇-Alkinylreste, die unsubstituiert oder gegebenenfalls mit OH, NR₅R₆ oder C₁-C₄-Alkoxyresten substituiert sind, C₁-C₂-Alkyl-C₃-C₅-cycloalkylreste, C(=O)R₇ bedeuten oder R₅ und R₆ unter Bildung eines Rings mit 5 oder 6 Atomen ausgewählt aus O, S, C oder N verbunden sein können, z.B. eines Pyrrolidin-, Oxazolidin-, Thiomorpholin-, Thiomorpholin-1,1-dioxid-, Morpholin-, Piperazin- oder Thiazolidin-1,1-dioxidrings, der unsubstituiert oder gegebenenfalls am Kohlenstoff mit OH, (=O), Halogen, C₁-C₄-Alkoxyresten, C(=O)R₇, C₁-C₆-Alkylresten, gegebenenfalls mit OH, Halogen, C₁-C₄-Alkoxyresten, C(=O)R₇ substituierten C₁-C₆-Alkylresten oder am Stickstoffatom mit C₁-C₄-Alkoxyresten, C(=O)R₇, S(=O)ₘR₈, C₁-C₆-Alkylresten oder gegebenenfalls mit OH, Halogen, C₁-C₄-Alkoxyresten, C(=O)R₇ substituierten C₂-C₆-Alkylresten oder am Schwefel mit (=O)ₘ, worin m 0 bis 2 ist, substituiert sein kann,
R₇ ein C₁-C₈-Alkylrest, ein gegebenenfalls mit OH, NR₅R₆, Halogen, C₁-C₄-Alkoxyresten oder C(=O)R₉ substituierter C₁-C₈-Alkylrest, C₁-C₄-Alkoxyrest, C₂-C₄-Alkoxyrest, der gegebenenfalls mit OH, NR₅R₆, Halogen oder C₁-C₄-Alkoxyresten substituiert ist, NR₅R₆ oder ein Phenylrest oder R₁₀ ist, von denen jeder unsubstituiert oder gegebenenfalls mit OH, Halogen, C₁-C₃-Alkylresten, C₁-C₃-Halogenalkoxyresten, (CH₂)ₙNR₅R₆, S(=O)ₘR₈ oder SO₂NR₅R₆ substituiert sein kann, wobei n 0 oder 1 ist und m 0 bis 2 ist,
R₈ ein C₁-C₄-Alkylrest, gegebenenfalls mit OH, NR₅R₆, Halogen, C₁-C₄-Alkoxyresten oder C(=O)R₇ substituierter C₂-C₄-Alkylrest ist,
R₉ ein C₁-C₄-Alkylrest, C₁-C₄-Alkoxyamino, C₁-C₃-Alkylamino- oder Di-C₁-C₃-alkylaminorest ist,
R₁₀ ein monocyclisches Ringsystem mit 5 oder 6 Atomen ist aufgebaut aus C, N, O und/oder S, z.B. Furan, Thiophen, Pyrrol, Pyrazol, Imidazol, Triazol, Tetrazol, Oxazol, Isoxazol, Isothiazol, Thiazol, Thiadiazol, Pyridin, Pyrimidin, Pyridazin und Pyrazin und
G C(=O) oder SO₂ ist oder worin
der Carboanhydrasehemmer (-)-trans-5,6-Dihydro-6-(3-methoxy)propyl-4-propylamino-4H-thieno-[2,3-b]thiopyran-2-sulfonamid-7,7-dioxid ist.

2. Zusammensetzung nach Anspruch 1, worin der Carboanhydrasehemmer ausgewählt ist aus der Gruppe bestehend aus (R)-3,4-Dihydro-2-(2-ethoxy)ethyl-4-ethylamino-2H-thieno-[3,2-e]-1,2-thiazin-6-sulfonamid-1,1-dioxid, (R)-3,4-Dihydro-2-(2-ethoxy)ethyl-4-propylamino-2H-thieno-[3,2-e]-1,2-thiazin-6-sulfonamid-1,1-dioxid, (R)-3,4-Dihydro-4-ethylamino-2-(3-methoxy)propyl-2H-thieno-[3,2-e]-1,2-thiazin-6-sulfonamid-1,1-dioxid, (R)-3,4-Dihydro-2-(3-methoxy)propyl-4-propylamino-2H-thieno-[3,2-e]-1,2-thiazin-6-sulfonamid-1,1-dioxid, (R)-3,4-Dihydro-4-ethylamino-2-[2-methoxyethoxy)ethyl]-2H-thieno-[3,2-e]-1,2-thiazin-6-sulfonamid-1,1-dioxid, (R)-3,4-Dihydro-2-[2-methoxyethoxy)ethyl]-4-propylamino-2H-thieno[3,2-e]-1,2-thiazin-6-sulfonamid-1,1-dioxid, (R)-3,4-Dihydro-4-ethylamino-2-[3-(2-methoxy)ethoxylpropyl-2H-thieno-[3,2-e]-1,2-thiazin-6-sulfonamid-1,1-dioxid, (R)-3,4-Dihydro-2-[3-(methoxyethoxy)propyl]-4-propylamino-2H-thieno-[3,2-e]-1,2-thiazin-6-sulfonamid-1,1-dioxid, (R)-3,4-Dihydro-4-ethylamino-2-(2-methoxy)ethyl-2H-thieno-[3,2-e]-1,2-thiazin-6-sulfonamid-1,1-dioxid, (R)-3,4-Dihydro-2-(2-methoxy)ethyl-4-propylamino-2H-thieno-[3,2-e]-1,2-thia zin-6-sulfonamid-1,1-dioxid, (R)-3,4-Dihydro-4-ethylamino-2-methyl-2H-thieno-[3,2-e]-1,2-thiazin-6-sulfonamid-1,1-dioxid, (R)-4-Ethylamino-3,4-dihydro-2-(4-methoxy)butyl-2H-thieno-[3,2-e]-1,2-thiazin-6-sulfonamid-1,1-dioxid, (R)-3,4-Dihydro-2-(4-methoxy)butyl-4-propylamino-2-thieno-[3,2-e]-1,2-thiazin-6-sulfonamid-1,1-dioxid, (R)-4-Ethylamino-2-(4-methoxyphenyl)-3,4-dihydro-2H-thieno-[3,2-e]-1,2-thiazin-6-sulfonamid-1,1-dioxid, (R)-4-Ethylamino-3,4-dihydro-2-(3-methoxyphenyl)-2H-thieno-[3,2-e]-1,2-thiazin-6-sulfonamid-1,1-dioxid, (R)-4-Ethylamino-2-(4-hydroxyphenyl)-3,4-dihydro-2H-thieno-[3,2-e]-1,2-thiazin-6-sulfonamid-1,1-dioxid, (R)-4-Ethylamino-3,4-dihydro-2-(3-hydroxyphenyl)-2H-thieno-[3,2-e]-1,2-thiazin-6-sulfonamid-1,1-dioxid, (R)-4-Ethylamino-3,4-dihydro-2-(3-hydroxyphenylmethyl)-2H-thieno-[3,2-e]-1,2-thiazin-6-sulfonamid-1,1-dioxid, (R)-4-Ethylamino-3,4-dihydro-2-(3-methoxyphenylmethyl)-2H-thieno-[3,2-e]-1,2-thiazin-6-sulfonamid-1,1-dioxid, (R)-4-Ethylamino-3,4-dihydro-2-(2-methylpropyl)-2H-thieno-[3,2-e]-1,2-thiazin-6-sulfonamid-1,1-dioxid, (R)-4-Ethylamino-3,4-dihydro-2-(6-hydroxyhexyl)-2H-thieno-[3,2-e]-1,2-thiazin-6-sulfonamid-1,1-dioxid, (R)-3,4-Dihydro-2-(3-hydroxypropyl)-4-(2-methylpropyl)amino-2H-thieno-[3,2-e]-1,2-thiazin-6-sulfonamid-1,1-dioxid und (-)-trans-5,6-Dihydro-6-(3-methoxy)propyl-4-propylamino-4H-thieno-[2,3-b]thiopyran-2-sulfonamid-7,7-dioxid.

3. Zusammensetzung nach Anspruch 2, worin der Carboanhydrasehemmer ausgewählt ist aus der Gruppe bestehend aus (R)-3,4-Dihydro-4-ethylamino-2-(3-methoxy)propyl-2H-thieno-[3,2-e]-1,2-thiazin-6-sulfonamid-1,1-dioxid und (R)-3,4-Dihydro-4-ethylamino-2-[2-methoxyethoxy)ethyl]-2H- thieno-[3,2-e]-1,2-thiazin-6-sulfonamid-1,1-dioxid, (R)-4-Ethylamino-3,4-dihydro-2-(4-methoxy)butyl-2H-thieno-[3,2-e]-1,2-thiazin-6-sulfonamid-1,1-dioxid, (R)-3,4-Dihydro-2-(4-methoxy)butyl-4-propylamino-2-thieno-[3,2-e]-1,2-thiazin-6-sulfonamid-1,1-dioxid, (-)-trans-5,6-Dihydro-6-(3-methoxy)propyl-4-propylamino-4H-thieno-[2,3-b]thiopyran-2-sulfonamid-7,7-dioxid.

4. Zusammensetzung nach Anspruch 1, worin der Carboanhydrasehemmer ausgewählt ist aus der Gruppe bestehend aus (R)-3,4-Dihydro-2-(2-ethoxy)ethyl-4-ethylamino-2H-thieno-[3,2-e]-1,2-thiazin-6-sulfonamid-1,1-dioxidhydrochlorid, (R)-3,4-Dihydro-2-(2-ethoxy)ethyl-4-propylamino-2H-thieno-[3,2-e]-1,2-thiazin-6-sulfonamid-1,1-dioxidhydrochlorid, (R)-3,4-Dihydro-4-ethylamino-2-(3-methoxy)propyl-2H-thieno-[3,2-e]-1,2-thiazin-6-sulfonamid-1,1-dioxidhydrochlorid, (R)-3,4-Dihydro-2-(3-methoxy)propyl-4-propylamino-2H-thieno-[3,2-e]-1,2-thiazin-6-sulfonamid-1,1-dioxidhydrochlorid, (R)-3,4-Dihydro-4-ethylamino-2-[2-methoxyethoxy)ethyl]-2H-thieno-[3,2-e]-1,2-thiazin-6-sulfonamid-1,1-dioxidhydrochlorid, (R)-3,4-Dihydro-2-[2-methoxy-ethoxy)ethyl]-4-propylamino-2H-thieno[3,2-e]-1,2-thiazin-6-sulfonamid-1,1-dioxidhydrochlorid, (R)-3,4-Dihydro-4-ethylamino-2-[3-(2-methoxy)ethoxylpropyl-2H-thieno-[3,2-e]-1,2-thiazin-6-sulfonamid-1,1-dioxidhydrochlorid, (R)-3,4-Dihydro-2-[3-(methoxyethoxy)propyl]-4-propylamino-2H-thieno-[3,2-e]-1,2-thiazin-6-sulfonamid-1,1-dioxidhydrochlorid, (R)-3,4-Dihydro-4-ethylamino-2-(2-methoxy)ethyl-2H-thieno-[3,2-e]-1,2-thiazin-6-sulfonamid-1,1-dioxidhydrochlorid, (R)-3,4-Dihydro-2-(2-methoxy)ethyl-4-propylamino-2H-thieno- [3,2-e]-1,2-thiazin-6-sulfonamid-1,1-dioxidhydrochlorid, (R)-3,4-Dihydro-4-ethylamino-2-methyl-2H-thieno-[3,2-e]-1,2-thiazin-6-sulfonamid-1,1-dioxidhydrochlorid, (R)-4-Ethylamino-3,4-dihydro-2-(4-methoxy)butyl-2H-thieno-[3,2-e]-1,2-thiazin-6-sulfonamid-1,1-dioxid, (R)-3,4-Dihydro-2-(4-methoxy)butyl-4-propylamino-2-thieno-[3,2-e]-1,2-thiazin-6-sulfonamid-1,1-dioxid, (R)-4-Ethylamino-2-(4-methoxyphenyl)-3,4-dihydro-2H-thieno-[3,2-e]-1,2-thiazin-6-sulfonamid-1,1-dioxidhydrochlorid, (R)-4-Ethylamino-3,4-dihydro-2-(3-methoxyphenyl)-2H-thieno-[3,2-e]-1,2-thiazin-6-sulfonamid-1,1-dioxidhydrochlorid, (R)-4-Ethylamino-2-(4-hydroxyphenyl)-3,4-dihydro-2H-thieno-[3,2-e]-1,2-thiazin-6-sulfonamid-1,1-dioxidhydrochlorid, (R)-4-Ethylamino-3,4-dihydro-2-(3-hydroxyphenyl)-2H-thieno-[3,2-e]-1,2-thiazin-6-sulfonamid-1,1-dioxidhydrochlorid, (R)-4-Ethylamino-3,4-dihydro-2-(3-hydroxyphenylmethyl)-2H-thieno-[3,2-e]-1,2-thiazin-6-sulfonamid-1,1-dioxidhydrochlorid, (R)-4-Ethylamino-3,4-dihydro-2-(3-methoxyphenylmethyl)-2H-thieno-[3,2-e]-1,2-thiazin-6-sulfonamid-1,1-dioxidhydrochlorid, (R)-4-Ethylamino-3,4-dihydro-2-(2-methylpropyl)-2H-thieno-[3,2-e]-1,2-thiazin-6-sulfonamid-1,1-dioxidhydrochlorid, (R)-4-Ethylamino-3,4-dihydro-2-(6-hydroxyhexyl)-2H-thieno-[3,2-e]-1,2-thiazin-6-sulfonamid-1,1-dioxidhydrochlorid, (R)-3,4-Dihydro-2-(3-hydroxypropyl)-4-(2-methylpropyl)amino-2H-thieno-[3,2-e]-1,2-thiazin-6-sulfonamid-1,1-dioxidhydrochlorid-hemihydrat und (-)-trans-5,6-Dihydro-6-(3-methoxy)propyl-4-propylamino-4H-thieno-[2,3-b]thiopyran-2-sulfonamid-7,7-dioxid.

5. Zusammensetzung nach Anspruch 4, worin der Carboanhydrasehemmer ausgewählt ist aus der Gruppe bestehend aus (R)-3,4-Dihydro-4-ethylamino-2-(3-methoxy)propyl-2H-thieno-[3,2-e]-1,2-thiazin-6-sulfonamid-1,1-dioxidhydrochlorid und (R)-3,4-Dihydro-4-ethylamino-2-(2-methoxy)ethyl-2H-thieno-[3,2-e]-1,2-thiazin-6-sulfonamid-1,1-dioxidhydrochlorid, (R)-4-Ethylamino-3,4-dihydro-2-(4-methoxy)butyl-2H-thieno-[3,2-e]-1,2-thiazin-6-sulfonamid-1,1-dioxid, (R)-3,4-Dihydro-2-(4-methoxy)butyl-4-propylamino-2-thieno-[3,2-e]-1,2-thiazin-6-sulfonamid-1,1-dioxid, (-)trans-5,6-Dihydro-6-(3-methoxy)propyl-4-propylamino-4H-thieno-[2,3-b]thiopyran-2-sulfonamid-7,7-dioxid.

6. Zusammensetzung nach einem der Ansprüche 1 bis 5, worin die Konzentration des Betablockers in der endgültigen Zusammensetzung ≤ etwa 2,0 Gew.-% ist und die Konzentration des Carboanhydrasehemmers in der endgültigen Zusammensetzung ≤ etwa 5 Gew.-% ist.

7. Zusammensetzung nach Anspruch 6, worin die Konzentration des Betablockers in der endgültigen Zusammensetzung etwa 0,1 bis etwa 1,0 Gew.-% ist.

8. Zusammensetzung nach Anspruch 7, worin die Konzentration des Betablockers in der endgültigen Zusammensetzung etwa 0,25 bis etwa 0,5 Gew.-% ist.

9. Zusammensetzung nach Anspruch 8, worin die Konzentration des Betablockers in der endgültigen Zusammensetzung 0,25 Gew.-% ist.

10. Zusammensetzung nach Anspruch 6, worin die Konzentration des Carboanhydrasehemmers in der endgültigen Zusammensetzung etwa 0,25 bis etwa 3 Gew.-% ist.

11. Zusammensetzung nach Anspruch 10, worin die Konzentration des Carboanhydrasehemmers in der endgültigen Zusammensetzung etwa 0,5 bis etwa 2 Gew.-% ist.

12. Zusammensetzung nach einem der Ansprüche 1 bis 11, worin der Betablocker ausgewählt ist aus den racemischen und enantiomeren Formen von Betaxolol, Timolol, Metoprolol, Befunolol, Falintolol, Levobunolol, Carteolol, Mepindolol, Pindolol, Bisoprolol, Bopindolol, Atenolol, Arotinolol, Acebutolol, Nadolol, Celiprolol, Metipranolol, Bevantolol, ICI 118 551, Pamatolol, Penbutolol, Toliprolol, Tiprenolol, Practolol, Procinolol, Exaprolol, Cicloprolol, Carazolol, Tazolol, Tienoxolol, Oxprenolol, Propranolol, IPS 339, Labetolol, Dilevalol, Esmolol, Bupranolol, Bunolol, Isoxaprolol, Diacetolol, Hydroxylevobunolol, Carvedilol und deren pharmazeutisch annehmbaren Salzen.

13. Zusammensetzung nach Anspruch 12, worin der Betablocker ausgewählt ist aus den racemischen und enantiomeren Formen von Betaxolol, Timolol, Carteolol, Levobunolol und Hydroxylevobunolol und deren pharmazeutisch annehmbaren Salzen.

14. Zusammensetzung nach Anspruch 13, worin der Betablocker Betaxolol oder ein pharmazeutisch annehmbares Salz davon ist.

15. Zusammensetzung nach Anspruch 13, worin der Betablocker S-Timolol oder ein pharmazeutisch annehmbares Salz davon ist.

16. Zusammensetzung nach einem der Ansprüche 1 bis 11, worin der Betablocker ein Thiadiazol der Formel 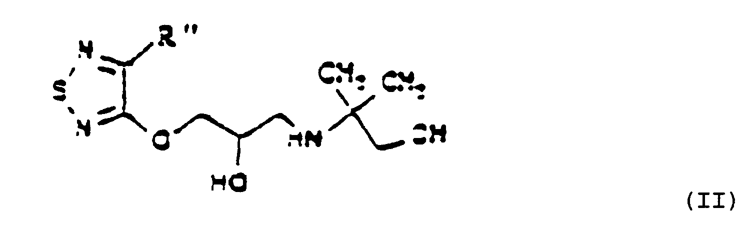 und ein optisch aktives Isomer oder pharmakologisch annehmbares Salz davon ist, worin R'' ausgewählt ist aus der Gruppe bestehend aus Wasserstoff, Halogen, C₁-C₅-Alkyl-, C₂-C₅-Monoalkenyl-, C₂-C₅-Alkoxy-, C₃-C₆-Cycloalkyl-, Phenyl-, Phenalkyl-, Morpholino-, Furyl-, Thienyl- und Pyrrylresten.

17. Zusammensetzung nach Anspruch 16, worin R'' ausgewählt ist aus der Gruppe bestehend aus Chlor, Ethyl-, Allyl-, Cyclopropyl-, Ethoxy-, Phenyl-, Phenylchlormethyl- und 2-(Cyclopropylmethoxy)ethylresten.

18. Zusammensetzung nach einem der Ansprüche 1 bis 17, weiter umfassend ein anionisches mukomimetisches Polymer, wobei die Konzentration des anionischen mukomimetischen Polymers in der endgültigen Zusammensetzung zwischen etwa 0,05 und etwa 8,0 Gew.-% liegt.

19. Zusammensetzung nach Anspruch 18, die weiterhin ein feinverteiltes Arzneimittelträger-Substrat umfaßt, wobei die Konzentration des feinverteilten Arzneimittelträger-Substrats in der endgültigen Zusammensetzung zwischen etwa 0,05 und etwa 10,0 Gew.-% liegt.

20. Zusammensetzung nach einem der Ansprüche 1 bis 19 zur Verwendung zur Behandlung von Glaukomen und okularem Hochdruck.

21. Verwendung einer Zusammensetzung umfassend einen Betablocker und einen Carboanhydrasehemmer in einem ophthalmologisch annehmbaren Träger, wobei die Konzentration des Betablockers in der endgültigen Zusammensetzung ≥ etwa 2,0 Gew.-% ist und die Konzentration des Carboanhydrasehemmers in der endgültigen Zusammensetzung ≤ 5 Gew.-% ist und wobei der Carboanhydrasehemmer die Formel hat, oder ein pharmazeutisch annehmbares Salz davon ist, worin
R₁ H, ein C₁-C₄-Alkylrest, C₂-C₄-Alkylrest ist, der gegebenenfalls mit OH, Halogen, C₁-C₄-Alkoxyresten oder C(=O)R₇ substituiert ist,
R₂ H, ein C₁-C₈-Alkylrest, C₂-C₈-Alkylrest, der mit OH, NR₅R₆, Halogen, C₁-C₄-Alkoxyresten, C₂-C₄-Alkoxy-C₁-C₄-alkoxyresten, OC(=O)R₇ oder C(=O)R₇ substituiert ist, C₃-C₇-Alkenylrest, der unsubstituiert oder gegebenenfalls mit OH, NR₅R₆ oder C₁-C₄-Alkoxyresten substituiert ist, C₃-C₇-Alkinylrest, der unsubstituiert oder gegebenenfalls mit OH, NR₅R₆ oder C₁-C₄-Alkoxyresten substituiert ist, C₁-C₃-Alkylrest, der mit Phenylresten oder R₁₀ substituiert ist, die jeweils unsubstituiert oder gegebenenfalls mit C₁-C₃-Alkylresten, C₁-C₃-Halogenalkylresten, OH, (CH₂)ₙNR₅R₆, Halogen, C₁-C₄-Alkoxyresten, C₁-C₄-Halogenalkoxyresten, C(=O)R₇, S(=O)ₘR₈ oder SO₂NR₅R₆ substituiert sein können, wobei m 0 bis 2 ist und n 0 bis 2 ist, C₂-C₄-Alkoxyrest, der gegebenenfalls mit NR₅R₆, Halogen, C₁-C₄-Alkoxyresten oder C(=O)R₇ substituiert ist, ein Phenylrest oder R₁₀ ist, die jeweils unsubstituiert oder gegebenenfalls mit C₁-C₃-Alkylresten, C₁-C₃-Halogenalkylresten, OH, (CH₂)ₙNR₅R₆, Halogen, C₁-C₄-Alkoxyresten, C₁-C₄-Halogenalkoxyresten, C(=O)R₇, S(=O)ₘR₈ oder SO₂NR₅R₆ substituiert sein können, worin m 0 bis 2 ist und n 0 bis 2 ist, mit dem Vorbehalt, daß R₁ und R₂ nicht beide H sein können, oder R₁ und R₂ verbunden sein können unter Bildung eines gesättigten Rings mit 5 oder 6 Atomen ausgewählt aus O, S, C oder N, z.B. eines Pyrrolidin-, Oxazolidin-, Thiomorpholin-, Thiomorpholin-1,1-dioxid-, Morpholin-, Piperazin-, Thiazolidin-1,1-dioxid- oder Tetrahydrooxazinrings, der unsubstituiert oder gegebenenfalls am Kohlenstoff mit OH, NR₅R₆, Halogen, C₁-C₄-Alkoxyresten, C(=O)R₇, C₁-C₆-Alkylresten, C₁-C₆-Alkylresten, die gegebenenfalls mit OH, NR₅R₆, Halogen-, C₁-C₄-Alkoxyresten, C(=O)R₇ oder am Stickstoff mit NR₅R₆, C₁-C₄-Alkoxyresten, C(=O)R₇, C₁-C₆-Alkylresten oder C₂-C₆-Alkylresten, die gegebenenfalls mit OH, NR₅R₆, Halogen, C₁-C₄-Alkoxyresten oder C(=O)R₇ substituiert sind, substituiert sein kann,
R₃ H, Halogen, ein C₁-C₄-Alkylrest, C₁-C₈-Alkoxyrest, C₁-C₈-Alkylthiolrest, C₂-C₈-Alkoxyrest, der gegebenenfalls mit OH, NR₅R₆, Halogen, C₁-C₄-Alkoxyresten oder C(=O)R₇ substituiert ist, ein gegebenenfalls mit R₄ substituierter C₁-C₄-Alkylrest ist oder worin R₁ und R₃ miteinander verbunden sein können zusammen mit Kohlenstoffatomen unter Bildung eines Rings mit 5 bis 7 Gliedern, wobei die Kohlenstoffatome unsubstituiert oder gegebenenfalls mit R₄ substituiert sein können,
R₄ OH, ein unsubstituierter oder gegebenenfalls mit OH, NR₅R₆, Halogen, C₁-C₄-Alkoxyresten oder C(=O)R₇ substituierter C₁-C₄-Alkylrest, C₁-C₄-Alkoxyrest, gegebenenfalls mit OH, NR₅R₆, Halogen, C₁-C₄-Alkoxyresten oder C(=O)R₇ substituierter C₂-C₄-Alkoxyrest, NR₅R₆, Phenylrest oder R₁₀ ist, von denen jeder unsubstituiert oder gegebenenfalls mit OH, (CH₂)ₙNR₅R₆, Halogen, C₁-C₄-Alkoxyresten, C₁-C₄-Halogenalkoxyresten, C(=O)R₇, S(=O)ₘR₈ oder SO₂NR₅R₆ substituiert sein kann, worin m 0 bis 2 ist und n 0 bis 2 ist,
mit dem Vorbehalt, daß dann, wenn G SO₂ ist und R₃ in Position 4 ist und H oder Halogen ist, daß dann R₁ und R₂ nicht H, gegebenenfalls mit OH substituierte C₁-C₆-Alkylreste, C₁-C₆-Alkoxyreste, C₂-C₆-Alkoxycarbonylreste, C₂-C₆-Alkenylreste, Phenyl-, Phenoxy-, Pyridyl-, Tetrahydrofuryl-, C₂-C₆-Alkanoyl-, C₂-C₆-Alkenylreste sind, noch unter Bildung eines 5-, 6- oder 7-gliedrigen Rings verbunden sind, der gesättigt oder ungesättigt sein kann und aus Atomen gegebenenfalls ausgewählt aus C, O, S und N aufgebaut ist, wobei der Stickstoff, wenn er gesättigt ist, gegebenenfalls mit H oder C₁-C₆-Alkylresten substituiert ist, oder worin der Kohlenstoff gegebenenfalls mit C₁-C₆-Alkyl-, C₁-C₆-Alkoxyresten oder OH substituiert ist und wenn R₃ in Position 5 ist und H, Cl, Br oder ein C₁-C₃-Alkylrest ist, daß dann weder R₁ noch R₂ H oder ein C₁-C₄-Alkylrest sein können und wenn G C(=O) ist und in Position 5 ist und R₃ H ist, daß dann R₁ und R₂ nicht beide CH₃ sein können;
R₅ und R₆ gleich oder verschieden sind und H, C₁-C₄-Alkylreste, gegebenenfalls mit OH, Halogen, C₁-C₄-Alkoxyresten oder C(=O)R₇ substituierte C₂-C₄-Alkylreste, C₁-C₄-Alkoxyreste, gegebenenfalls mit OH, Halogen, C₁-C₄-Alkoxyresten oder C(=O)R₇ substituierte C₂-C₄-Alkoxyreste, C₃-C₇-Alkenylreste, die unsubstituiert oder gegebenenfalls mit OH, NR₅R₆ oder C₁-C₄-Alkoxyresten substituiert sind, C₃-C₇-Alkinylreste, die unsubstituiert oder gegebenenfalls mit OH, NR₅R₆ oder C₁-C₄-Alkoxyresten substituiert sind, C₁-C₂-Alkyl-C₃-C₅-cycloalkylreste, C(=O)R₇ bedeuten oder R₅ und R₆ unter Bildung eines Rings mit 5 oder 6 Atomen ausgewählt aus O, S, C oder N verbunden sein können, z.B. eines Pyrrolidin-, Oxazolidin-, Thiomorpholin-, Thiomorpholin-1,1-dioxid-, Morpholin-, Piperazin- oder Thiazolidin-1,1-dioxidrings, der unsubstituiert oder gegebenenfalls am Kohlenstoff mit OH, (=O), Halogen, C₁-C₄-Alkoxyresten, C(=O)R₇, C₁-C₆-Alkylresten, gegebenenfalls mit OH, Halogen, C₁-C₄-Alkoxyresten, C(=O)R₇ substituierten C₁-C₆-Alkylresten oder am Stickstoffatom mit C₁-C₄-Alkoxyresten, C(=O)R₇, S(=O)ₘR₈, C₁-C₆-Alkylresten oder gegebenenfalls mit OH, Halogen, C₁-C₄-Alkoxyresten, C(=O)R₇ substituierten C₂-C₆-Alkylresten oder am Schwefel mit (=O)m, worin m 0 bis 2 ist, substituiert sein kann,
R₇ ein C₁-C₈-Alkylrest, ein gegebenenfalls mit OH, NR₅R₆, Halogen, C₁-C₄-Alkoxyresten oder C(=O)R₉ substituierter C₁-C₈-Alkylrest, C₁-C₄-Alkoxyrest, C₂-C₄-Alkoxyrest, der gegebenenfalls mit OH, NR₅R₆, Halogen oder C₁-C₄-Alkoxyresten substituiert ist, NR₅R₆ oder ein Phenylrest oder R₁₀ ist, von denen jeder unsubstituiert oder gegebenenfalls mit OH, Halogen, C₁-C₃-Alkylresten, C₁-C₃-Halogenalkoxyresten, (CH₂)ₙNR₅R₆, S(=O)ₘR₈ oder SO₂NR₅R₆ substituiert sein kann, wobei n 0 oder 1 ist und m 0 bis 2 ist,
R₈ ein C₁-C₄-Alkylrest, gegebenenfalls mit OH, NR₅R₆, Halogen, C₁-C₄-Alkoxyresten oder C(=O)R₇ substituierter C₂-C₄-Alkylrest ist,
R₉ ein C₁-C₄-Alkylrest, C₁-C₄-Alkoxyamino-, C₁-C₃-Alkylamino- oder Di-C₁-C₃-alkylaminorest ist,
R₁₀ ein monocyclisches Ringsystem mit 5 oder 6 Atomen ist aufgebaut aus C, N, O und/oder S, z.B. Furan, Thiophen, Pyrrol, Pyrazol, Imidazol, Triazol, Tetrazol, Oxazol, Isoxazol, Isothiazol, Thiazol, Thiadiazol, Pyridin, Pyrimidin, Pyridazin und Pyrazin und
G C(=O) oder SO₂ ist oder worin
der Carboanhydrasehemmer (-)-trans-5,6-Dihydro-6-(3-methoxy)propyl-4-propylamino-4H-thieno-[2,3-b]thiopyran-2-sulfonamid-7,7-dioxid ist, zur Herstellung eines Arzneimittels zur Behandlung von Glaukomen und Augenhochdruck.

22. Verwendung nach Anspruch 21, worin der Carboanhydrasehemmer ausgewählt ist aus der Gruppe bestehend aus (R)-3,4-Dihydro-2-(2-ethoxy)ethyl-4-ethylamino-2H-thieno-[3,2-e]-1,2-thiazin-6-sulfonamid-1,1-dioxid, (R)-3,4-Dihydro-2-(2-ethoxy)ethyl-4-propylamino-2H-thieno-[3,2-e]-1,2-thiazin-6-sulfonamid-1,1-dioxid, (R)-3,4-Dihydro-4-ethylamino-2-(3-methoxy)propyl-2H-thieno-[3,2-e]-1,2-thiazin-6-sulfonamid-1,1-dioxid, (R)-3,4-Dihydro-2-(3-methoxy)propyl-4-propylamino-2H-thieno-[3,2-e]-1,2-thia zin-6-sulfonamid-1,1-dioxid, (R)-3,4-Dihydro-4-ethylamino-2-[2-methoxyethoxy)ethyl]-2H-thieno-[3,2-e]-1,2-thiazin-6-sulfonamid-1,1-dioxid, (R)-3,4-Dihydro-2-[2-methoxyethoxy)ethyl]-4-propylamino-2H-thieno[3,2-e]-1,2-thiazin-6-sulfonamid-1,1-dioxid, (R)-3,4-Dihydro-4-ethylamino-2-[3-(2-methoxy)ethoxy]propyl-2H-thieno-[3,2-e]-1,2-thiazin-6-sulfonamid-1,1-dioxid, (R)-3,4-Dihydro-2-[3-(methoxyethoxy)propyl]-4-propylamino-2H-thieno-[3,2-e]-1,2-thiazin-6-sulfonamid-1,1-dioxid, (R)-3,4-Dihydro-4-ethylamino-2-(2-methoxy)ethyl-2H-thieno-[3,2-e]-1,2-thiazin-6-sulfonamid-1,1-dioxid, (R)-3,4-Dihydro-2-(2-methoxy)ethyl-4-propylamino-2H-thieno-[3,2-e]-1,2-thiazin-6-sulfonamid-1,1-dioxid, (R)-3,4-Dihydro-4-ethylamino-2-methyl-2H-thieno-[3,2-e]-1,2-thiazin-6-sulfonamid-1,1-dioxid, (R)-4-Ethylamino-3,4-dihydro-2-(4-methoxy)butyl-2H-thieno-[3,2-e]-1,2-thiazin-6-sulfonamid-1,1-dioxid, (R)-3,4-Dihydro-2-(4-methoxy)butyl-4-propylamino-2-thieno-[3,2-e]-1,2-thiazin-6-sulfonamid-1,1-dioxid, (R)-4-Ethylamino-2-(4-methoxyphenyl)-3,4-dihydro-2H-thieno-[3,2-e]-1,2-thiazin-6-sulfonamid-1,1-dioxid, (R)-4-Ethylamino-3,4-dihydro-2-(3-methoxyphenyl)-2H-thieno-[3,2-e]-1,2-thiazin-6-sulfonamid-1,1-dioxid, (R)-4-Ethylamino-2-(4-hydroxyphenyl)-3,4-dihydro-2H-thieno-[3,2-e]-1,2-thiazin-6-sulfonamid-1,1-dioxid, (R)-4-Ethylamino-3,4-dihydro-2-(3-hydroxyphenyl)-2H-thieno-[3,2-e]-1,2-thiazin-6-sulfonamid-1,1-dioxid, (R)-4-Ethylamino-3,4-dihydro-2-(3-hydroxyphenylmethyl)-2H-thieno-[3,2-e]-1,2-thiazin-6-sulfonamid-1,1-dioxid, (R)-4-Ethylamino-3,4-dihydro-2-(3-methoxyphenylmethyl)-2H-thieno-[3,2-e]-1,2-thiazin-6-sulfonamid-1,1-dioxid, (R)-4-Ethylamino-3,4-dihydro-2-(2-methylpropyl)-2H-thieno- [3,2-e]-1,2-thiazin-6-sulfonamid-1,1-dioxid, (R)-4-Ethylamino-3,4-dihydro-2-(6-hydroxyhexyl)-2H-thieno-[3,2-e]-1,2-thiazin-6-sulfonamid-1,1-dioxid, (R)-3,4-Dihydro-2-(3-hydroxypropyl)-4-(2-methylpropyl) amino-2H-thieno-[3,2-e]-1,2-thiazin-6-sulfonamid-1,1dioxid und (-)-trans-5,6-Dihydro-6-(3-methoxy)propyl-4-propylamino-4H-thieno-[2, 3-b] thiopyran-2-sulfonamid-7,7-dioxid.

23. Verwendung nach Anspruch 21, worin der Carboanhydrasehemmer ausgewählt ist aus der Gruppe bestehend aus (R)-3,4-Dihydro-4-ethylamino-2-(3-methoxy)propyl-2H-thieno-[3,2-e]-1,2-thiazin-6-sulfonamid-1,1-dioxid und (R)-3,4-Dihydro-4-ethylamino-2-(2-methoxy)ethyl-2H-thieno-[3,2-e]-1,2-thiazin-6-sulfonamid-1,1-dioxid, (R)-4-Ethylamino-3,4-dihydro-2-(4-methoxy)butyl-2H-thieno-[3,2-e]-1,2-thiazin-6-sulfonamid-1,1-dioxid, (R)-3,4-Dihydro-2-(4-methoxy)butyl-4-propylamino-2-thieno-[3,2-e]-1,2-thiazin-6-sulfonamid-1,1-dioxid, (-)-trans-5,6-Dihydro-6-(3-methoxy)propyl-4-propylamino-4H-thieno-[2,3-b]-thiopyran-2-sulfonamid-7,7-dioxid.

24. Verwendung nach Anspruch 21, worin der Carboanhydrasehemmer ausgewählt ist aus der Gruppe bestehend aus (R)-3,4-Dihydro-2-(2-ethoxy)ethyl-4-ethylamino-2H-thieno-[3,2-e]-1,2-thiazin-6-sulfonamid-1,1-dioxidhydrochlorid, (R)-3,4-Dihydro-2-(2-ethoxy)ethyl-4-propylamino-2H-thieno-[3,2-e]-1,2-thiazin-6-sulfonamid-1,1-dioxidhydrochlorid, (R)-3,4-Dihydro-4-ethylamino-2-(3-methoxy)propyl-2H-thieno-[3,2-e]-1,2-thiazin-6-sulfonamid-1,1-dioxidhydrochlorid, (R)-3,4-Dihydro-2-(3-methoxy)propyl-4-propylamino-2H-thieno-[3,2-e]-1,2-thiazin-6-sulfonamid-1,1-dioxidhydrochlorid, (R)-3,4-Dihydro-4-ethylamino-2-[2-methoxyethoxy)ethyl]-2H-thieno-[3,2-e]-1,2-thiazin-6- sulfonamid-1,1-dioxidhydrochlorid, (R)-3,4-Dihydro-2-[2-methoxy-ethoxy)ethyl]-4-propylamino-2H-thieno[3,2-e]-1,2-thiazin-6-sulfonamid-1,1-dioxidhydrochlorid, (R)-3,4-Dihydro-4-ethylamino-2-[3-(2-methoxy)ethoxy]propyl-2H-thieno-[3,2-e]-1,2-thiazin-6-sulfonamid-1,1-dioxidhydrochlorid, (R)-3,4-Dihydro-2-[3-(methoxyethoxy)propyl]-4-propylamino-2H-thieno-[3,2-e]-1,2-thiazin-6-sulfonamid-1,1-dioxidhydrochlorid, (R)-3,4-Dihydro-4-ethylamino-2-(2-methoxy)ethyl-2H-thieno-[3,2-e]-1,2-thiazin-6-sulfonamid-1,1-dioxidhydrochlorid, (R)-3,4-Dihydro-2-(2-methoxy)ethyl-4-propylamino-2H-thieno-[3,2-e]-1,2-thiazin-6-sulfonamid-1,1-dioxidhydrochlorid, (R)-3,4-Dihydro-4-ethylamino-2-methyl-2H-thieno-[3,2-e]-1,2-thiazin-6-sulfonamid-1,1-dioxidhydrochlorid, (R)-4-Ethylamino-3,4-dihydro-2-(4-methoxy)butyl-2H-thieno-[3,2-e]-1,2-thiazin-6-sulfonamid-1,1-dioxid, (R)-3,4-Dihydro-2-(4-methoxy)butyl-4-propylamino-2-thieno-[3,2-e]-1,2-thiazin-6-sulfonamid-1,1-dioxid, (R)-4-Ethylamino-2-(4-methoxyphenyl)-3,4-dihydro-2H-thieno-[3,2-e]-1,2-thiazin-6-sulfonamid-1,1-dioxidhydrochlorid, (R)-4-Ethylamino-3,4-dihydro-2-(3-methoxyphenyl)-2H-thieno-[3,2-e]-1,2-thiazin-6-sulfonamid-1,1-dioxidhydrochlorid, (R)-4-Ethylamino-2-(4-hydroxyphenyl)-3,4-dihydro-2H-thieno-[3,2-e]-1,2-thiazin-6-sulfonamid-1,1-dioxidhydrochlorid, (R)-4-Ethylamino-3,4-dihydro-2-(3-hydroxyphenyl)-2H-thieno-[3,2-e]-1,2-thiazin-6-sulfonamid-1,1-dioxidhydrochlorid, (R)-4-Ethylamino-3,4-dihydro-2-(3-hydroxyphenylmethyl)-2H-thieno-[3,2-e]-1,2-thiazin-6-sulfonamid-1,1-dioxidhydrochlorid, (R)-4-Ethylamino-3,4-dihydro-2-(3-methoxyphenylmethyl)-2H-thieno-[3,2-e]-1,2-thiazin-6-sulfonamid-1,1-dioxidhydrochlorid, (R)-4-Ethylamino-3,4-dihydro-2-(2-methylpropyl)-2H-thieno-[3,2-e]-1,2-thiazin- 6-sulfonamid-1,1-dioxidhydrochlorid, (R)-4-Ethylamino-3,4-dihydro-2-(6-hydroxyhexyl)-2H-thieno-[3,2-e]-1,2-thiazin-6-sulfonamid-1,1-dioxidhydrochlorid, (R)-3,4-Dihydro-2-(3-hydroxypropyl)-4-(2-methylpropyl)amino-2H-thieno-[3,2-e]-1,2-thiazin-6-sulfonamid-1,1-dioxidhydrochlorid-hemihydrat und (-)-trans-5,6-Dihydro-6-(3-methoxy)propyl-4-propylamino-4H-thieno-[2,3-b]thiopyran2-sulfonamid-7,7-dioxid.

25. Verwendung nach Anspruch 21, worin der Carboanhydrasehemmer ausgewählt ist aus der Gruppe bestehend aus (R)-3,4-Dihydro-4-ethylamino-2-(3-methoxy)propyl-2H-thieno-[3,2-e]-1,2-thiazin-6-sulfonamid-1,1-dioxidhydrochlorid und (R)-3,4-Dihydro-4-ethylamino-2-(2-methoxy)ethyl-2H-thieno-[3,2-e]-1,2-thiazin-6-sulfonamid-1,1-dioxidhydrochlorid, (R)-4-Ethylamino-3,4-dihydro-2-(4-methoxy)butyl-2H-thieno-[3,2-e]-1,2-thiazin-6-sulfonamid-1,1-dioxid, (R)-3,4-Dihydro-2-(4-methoxy)butyl-4-propylamino-2-thieno-[3,2-e]-1,2-thiazin-6-sulfonamid-1,1-dioxid, (-)-trans-5,6-Dihydro-6-(3-methoxy)propyl-4-propylamino-4H-thieno-[2,3-b]thiopyran-2-sulfonamid-7,7-dioxid.

26. Verwendung nach einem der Ansprüche 21 bis 25, worin der Betablocker ausgewählt ist aus den racemischen und enantiomeren Formen von Betaxolol, Timolol, Metoprolol, Befunolol, Falintolol, Levobunolol, Carteolol, Mepindolol, Pindolol, Bisoprolol, Bopindolol, Atenolol, Arotinolol, Acebutolol, Nadolol, Celiprolol, Metipranolol, Bevantolol, ICI 118 551, Pamatolol, Penbutolol, Toliprolol, Tiprenolol, Practolol, Procinolol, Exaprolol, Cicloprolol, Carazolol, Tazolol, Tienoxolol, Oxprenolol, Propranolol, IPS 339, Labetolol, Dilevalol, Esmolol, Bupranolol, Bunolol, Isoxaprolol, Diacetolol und Hyroxylevobunolol und deren pharmazeutisch annehmbaren Salzen.

27. Verwendung nach Anspruch 26, worin der Betablocker ausgewählt ist aus der Gruppe bestehend aus Betaxolol, Timolol, Carteolol, Levobunolol und Hydroxylevobunolol und deren pharmazeutisch annehmbaren Salzen.

28. Verwendung nach einem der Ansprüche 21 bis 27, worin der Betablocker ein Thiadiazol der Formel 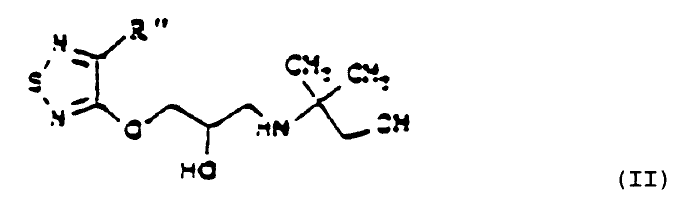 oder ein optisch aktives Isomer oder pharmakologisch annehmbares Salz davon ist, worin R'' ausgewählt ist aus der Gruppe bestehend aus Wasserstoff, Halogen, C₁-C₅-Alkyl-, C₂-C₅-Monoalkenyl-, C₂-C₅-Alkoxy-, C₃-C₆-Cycloalkyl-, Phenyl-, Phenalkyl-, Morpholino-, Furyl-, Thienyl- und Pyrrylresten.

29. Verwendung nach Anspruch 28, worin R'' ausgewählt ist aus der Gruppe bestehend aus Chlor, Ethyl-, Allyl-, Cyclopropyl-, Ethoxy-, Phenyl-, Phenylchlormethyl- und 2-(Cyclopropylmethoxy)ethylresten.

## Revendications

1. Composition ophtalmique topique, qui comprend un bêta-bloqueur et un inhibiteur d'anhydrase carbonique dans un véhicule acceptable du point de vue ophtalmique, où la composition est une suspension et le pH de la composition finale est compris entre environ 5,0 et environ 7,9 et l'inhibiteur d'anhydrase carbonique répond à la formule suivante : 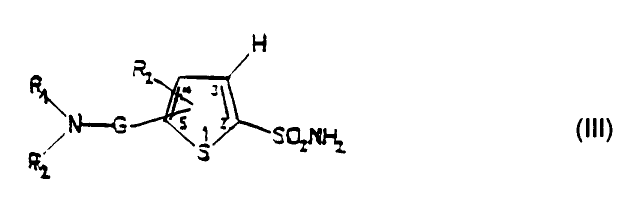 ou un sel pharmaceutiquement acceptable d'un tel composé, structure dans laquelle :
R₁ représente un atome d'hydrogène; un radical alkyle en C₁ à C₄, alkyle en C₂ à C₄ éventuellement substitué par OH, un atome d'halogène, un radical alcoxy en C₁ à C₄ ou C(=O)R₇;
R₂ représente un atome d'hydrogène, un radical alkyle en C₁ à C₈, un radical alkyle en C₂ à C₈ substitué par un OH, un radical NR₅R₆, un atome d'halogène, un radical alcoxy en C₁ à C₄, un radical alcoxy(C₂-C₄)alcoxy(C₁-C₄), un radical OC(=O)R₇ ou C(=O)R₇, un radical alcényle en C₃ à C₇ non substitué ou éventuellement substitué par OH, NR₅R₆, ou alcoxy en C₁ à C₄; un radical alcynyle en C₃ à C₇ non substitué ou éventuellement substitué par OH, NR₅R₆, ou alcoxy en C₁ à C₄; un radical alkyle en C₁ à C₃ substitué par un radical phényle ou R₁₀, chacun d'entre eux pouvant être non substitué ou éventuellement substitué par un radical alkyle en C₁ à C₃, haloalkyle en C₁ à C₃, OH, (CH₂)ₙNR₅R₆, un atome d'halogène, un radical alcoxy en C₁ à C₄, haloalcoxy en C₁ à C₄, C(=O)R₇, S(=O)ₙR₈ ou SO₂NR₅R₆, où m est égal à 0-2 et n est égal à 0-2; un radical alcoxy en C₁ à C₄ éventuellement substitué par NR₅R₆, un atome d'halogène, un radical alcoxy en C₁ à C₄ ou C(=O)R₇, un radical phényle ou R₁₀, chacun d'entre eux pouvant ne pas être substitué ou être substitué éventuellement par un radical alkyle en C₁ à C₃, haloalkyle en C₁ à C₃, OH, (CH₂)ₙNR₅R₆, un atome d'halogène, un radical alcoxy en C₁ à C₄, haloalcoxy en C₁ à C₄, C(=O)R₇, S(=O)ₘR₈ ou SO₂NR₅R₆, où m est 0-2 et n est 0-2, avec la condition que R₁ et R₂ ne représentent pas tous deux des atomes d'hydrogène; ou bien R₁ et R₂ peuvent être réunis pour former un noyau saturé comportant 5 ou 6 atomes choisis parmi O, S, C ou N, tels que, par exemple, un noyau pyrrolidine, oxazolidine, thiomorpholine, thiomorpholine-1,1-dioxyde, morpholine, pipérazine, thiazolidine, 1,1-dioxyde ou tétrahydrooxazine, qui peut ne pas être substitué ou être substitué éventuellement sur du carbone par OH, NR₅R₆, un atome d'halogène, un radical alcoxy en C₁ à C₄, C(=O)R₇, un radical alkyle en C₁ à C₆, un radical alkyle en C₁à C₆ éventuellement substitué par OH, NR₅R₆, un atome d'halogène, un radical alcoxy en C₁ à C₄, C(=O)R₇ ou sur l'azote par NR₅R₆, un radical alcoxy en C₁ à C₄, C(=O)R₇, un radical alkyle en C₁ à C₆ ou alkyle en C₂ à C₆ éventuellement substitué par OH, NR₅R₆, un atome d'halogène, un radical alcoxy en C₁ à C₄ ou C(=O)R₇;
R₃ représente un atome d'hydrogène; un atome d'halogène; un radical alkyle en C₁ à C₄; alcoxy en C₁ à C₈; alkylthiol en C₁ à C₈; alcoxy en C₂ à C₈ éventuellement substitué par OH, NR₅R₆, un atome d'halogène, un radical alcoxy en C₁ à C₄ ou C(=O)R₇; un radical alkyle en C₁ à C₄ éventuellement substitué par R₁; ou bien R₁ et R₃ peuvent être mutuellement liés à des atomes de carbone pour former un cycle à 5 à 7 chaînons dans lequel lesdits atomes de carbone peuvent ne pas être substitués ou être éventuellement substitués par R₄;
R₄ représente un groupe OH; un radical alkyle en C₁ à C₄ non substitué ou éventuellement substitué par OH, NR₅R₆, un atome d'halogène, un radical alcoxy en C₁ à C₄ ou C(=O)R₇; un radical alcoxy en C₁ à C₄; un radical alcoxy en C₂ à C₄ éventuellement substitué par OH, NR₅R₆, un atome d'halogène, un radical alcoxy en C₁ à C₄ ou C(=O)R₇; NR₅R₆; un radical phényle ou R₁₀, chacun d'entre eux n'étant pas substitué ou étant substitué éventuellement par OH, (CH₂)ₙNR₅R₆, un atome d'halogène, un radical alcoxy en C₁ à C₄, haloalcoxy en C₁ à C₄, C(=O)R₇, S(=O)ₘR₆ ou SO₂NR₅R₆, où m est 0-2 et n est 0-2;
avec la condition que lorsque G est SO₂ et que R₃ se trouve en position 4 et représente un atome d'hydrogène ou un atome d'halogène, R₁ et R₂ ne représentent pas d'atomes d'halogène, de radicaux alkyle en C₁ à C₆ éventuellement substitués par OH, de radical alcoxy en C₁ à C₆, de radical alcoxycarbonyle en C₂ à C₆, de radical alcényle en C₂ à C₆, de radical phényle, phénoxy, pyridyle, tétrahydrofuryle, de radical alcanoyle en C₂,à C₆, de radical alcényle en C₂ à C₆, ni qu'ils soient unis pour former un noyau à 5, 6 ou 7 chaînons, saturé ou insaturé, constitué d'atomes choisis parmi C, O, S, N, où ledit azote, lorsqu'il est saturé, est éventuellement substitué par H ou un radical alkyle en C₁ à C₆ ou dans lequel ledit carbone est éventuellement substitué par un radical alkyle en C₁ à C₆, alcoxy en C₁ à C₆ ou OH; et que lorsque R₃ se trouve dans la position 5 et est H, Cl, Br ou alkyle en C₁ à C₃, ni R₁ ni R₂ puissent être de l'hydrogène ou des radicaux alkyle en C₁ à C₄; et que lorsque G est C(=O) et se trouve en position 5 et R₃ représente H, alors ni R₁ ni R₂ ne puissent représenter tous deux CH₃;
R₅ et R₆ sont identiques ou différents et représentent un atome d'hydrogène; un radical alkyle en C₁ à C₄; alkyle en C₂ à C₄ éventuellement substitué par OH, un atome d'halogène, un radical alcoxy en C₁ à C₄ ou C(=O)R₇; un radical alcoxy en C₁ à C₄; un radical alcoxy en C₂ à C₄ éventuellement substitué par un groupe OH, un atome d'halogène, un radical alcoxy en C₁ à C₄ ou C(=O)R₇; un radical alcényle en C₃ à C₇ non substitué ou éventuellement substitué par OH, NR₅R₆, ou un radical alcoxy en C₁ à C₄; un radical alcynyle en C₃ à C₇ non substitué ou éventuellement substitué par OH, NR₅R₆, ou un radical alcoxy en C₁ à C₄; le radical alkyl(C₁-C₂)cycloalkyle(C₃-C₅); C(=O)R₇ ou bien R₅ et R₆ peuvent être unis pour former un noyau comportant 5 ou 6 atomes choisis parmi O, S, C ou N, comme un noyau pyrrolidine, oxazolidine, thiomorpholine, thiomorpholine-1,1-dioxyde, morpholine, pipérazine ou thiazolidine-1,1-dioxyde, qui peut ne pas être substitué ou être éventuellement substitué sur un atome de carbone par un groupe OH, (=O), un atome d'halogène, un radical alcoxy en C₁ à C₄, C(=O)R₇, un radical alkyle en C₁ à C₆, un radical alkyle C₁ à C₆ éventuellement substitué par un groupe OH, un atome d'halogène, un radical alcoxy en C₁ à C₄, C(=O)R₇, ou sur l'atome d'azote par un radical alcoxy en C₁ à C₄, C(=O)R₇, S(=O)ₙR₉, un radical alkyle en C₁ à C₆ ou un radical alkyle en C₂ à C₆ éventuellement substitué par un groupe OH, un atome d'halogène, un radical alcoxy en C₁ à C₄, C(=O)R₇, ou sur l'atome de soufre par un groupe (=O)ₘ, où m est 0-2;
R₇ représente un groupe alkyle en C₁ à C₈; un radical alkyle en C₁ à C₆ éventuellement substitué par un groupe OH, NR₅R₆, un atome d'halogène, un radical alcoxy en C₁ à C₄ ou C(=O)R₉; un radical alcoxy en C₁ à C₄; un radical alcoxy en C₂ à C₄ éventuellement substitué par un groupe OH, NR₅R₆, un atome d'halogène ou un radical alcoxy en C₁ à C₄, NR₅R₆, ou un radical phényle ou R₁₀, chacun d'entre eux pouvant ne pas être substitué ou être substitué éventuellement par un groupe OH, un atome d'halogène, un radical alkyle en C₁ à C₃, haloalcoxy en C₁ à C₃, (CH₂)ₙNR₅R₆, S(=O)ₘR₉ ou SO₂NR₅R₆, où n est égal à 0 ou à 1 et m est égal à 0-2;
R₈ représente un groupe alkyle en C₁ à C₄; un groupe alkyle en C₂ à C₄ éventuellement substitué par OH, NR₅R₆, un atome d'halogène, un radical alcoxy en C₁ à C₄ ou C(=O)R₇;
R₉ représente un radical alkyle en C₁ à C₄, un radical alcoxy en C₁ à C₄; un radical amino, un radical alkylamino en C₁ à C₃, ou un radical dialkylamino en C₁ à C₃;
R₁₀ représente un système à noyau monocyclique de 5 ou 6 atomes composé de C, N, O et/ou S, comme le furanne, le thiophène, le pyrrole, le pyrazole, l'imidazole, le triazole, le tétrazole, l'oxazole, l'isoxazole, l'isothiazole, le thiazole, le thiadiazole, la pyridine, la pyrimidine, la pyridazine et la pyrazine; et
G représente C(=O) ou SO₁, ou bien
où l'inhibiteur d'anhydrase carbonique est le (-)-trans-5,6-dihydro-6-(3-méthoxy)propyl-4-propylamino-4H-thiéno[2,3-b]-thiopyranne-2-sulfonamide 7,7-dioxyde

2. Composition suivant la revendication 1, caractérisée en ce que l'inhibiteur d'anhydrase carbonique est choisi dans le groupe formé par les composés qui suivent :
(R)-3,4-dihydro-2-(2-éthoxy)éthyl-4-éthylamino-2H-thiéno[3,2-e]-1,2-thiazine-6-sulfonamide 1,1 dioxyde; (R)-3,4-dihydro-2-(2-éthoxy)éthyl-4-propylamino-2H-thiéno[3,2-e]-1,2-thiazine-6-sulfonamide 1,1 dioxyde; (R)-3,4-dihydro-4-éthylamino-2-(3-méthoxy)propyl-2H-thiéno[3,2-e]-1,2-thiazine-6-sulfonamide 1,1-dioxyde; (R)-3,4-dihydro-2-(3-méthoxy)propyl-4-propylamino-2H-thiéno[3,2-e]-1,2-thiazine-6-sulfonamide 1,1-dioxyde; (R)-3,4-dihydro-4-éthylamino-2-[2-méthoxyéthoxy)éthyl]-2H-thiéno[3,2-e]-1,2-thiazine-6-sulfonamide 1,1-dioxyde; (R)-3,4-dihydro-2-[2-méthoxyéthoxy)éthyl]-4-propylamino-2H-thiéno-[3,2-e]-1,2-thiazine-6-sulfonamide 1,1-dioxyde; (R)-3,4-dihydro-4-éthylamino-2-[3-méthoxy)éthoxy)propyl]-2H-thiéno-[3,2-e]-1,2-thiazine-6-sulfonamide 1,1-dioxyde; (R)-3,4-dihydro-2-[3-(méthoxyéthoxy)propyl]-4-propylamino-2H-thiéno-[3,2-e]-1,2-thiazine-6-sulfonamide 1,1-dioxyde; (R)-3,4-dihydro-4-éthylamino-2-(2-méthoxy)éthyl-2H-thiéno[3,2-e]-1,2-thiazine-6-sulfonamide 1,1-dioxyde; (R)-3,4-dihydro-2-(2-méthoxy)éthyl-4-propylamino-2H-thiéno[3,2-e]-1,2-thiazine-6-sulfonamide 1,1-dioxyde; (R)-3,4-dihydro-4-éthylamino-2-méthyl-2H-thiéno[3,2-e]-1,2-thiazine-6-sulfonamide 1,1-dioxyde; (R)-4-éthylamino-3,4-dihydro-2-(4-méthoxy)butyl-2H-thiéno[3,2-e]-1,2-thiazine-6-sulfonamide 1,1-dioxyde; (R)-3,4-dihydro-2-(4-(méthoxy)butyl-4-propylamino-2-thiéno[3,2-e]-1,2-thiazine-6-sulfonamide 1,1-dioxyde; (R)-4-éthylamino-2-(4-méthoxyphényl)-3,4-dihydro-2H-thiéno[3,2-e]-1,2-thiazine-6-sulfonamide 1,1-dioxyde; (R)-4-éthylamino-3,4-dihydro-2-(3-méthoxyphényl)-2H-thiéno[3,2-e]-1,2-thiazine-6-sulfonamide 1,1-dioxyde; (R)-4-éthylamino-2-(4-hydroxyphényl)-3,4-dihydro-2H-thiéno[3,2-e]-1,2-thiazine-6-sulfonamide 1,1-dioxyde; (R)-4-éthylamino-3,4-dihydro-2-(3-hydroxyphényl)-2H-thiéno[3,2-e]1,2-thiazine-6-sulfonamide 1,1-dioxyde; (R)-4-éthylamino-3,4-dihydro-2-(3-hydroxyphényméthyl)-2H-thiéno-[3,2-e]-1,2-thiazine-6-sulfonamide 1,1-dioxyde; (R)-4-éthylamino-3,4-dihydro-2-(3-méthoxyphénylméthyl)-2H-thiéno-[3,2-e]-1,2-thiazine-6-sulfonamide 1,1-dioxyde; (R)-4-éthylamino-3,4-dihydro-2-(2-méthylpropyl)-2H-thiéno[3,2-e]-1,2-thiazine-6-sulfonamide 1,1-dioxyde; (R)-4-éthylamino-3,4-dihydro-2-(6-hydroxyhexyl)-2H-thiéno[3,2-e]-1,2-thiazine-6-sulfonamide 1,1-dioxyde; (R)-3,4-dihydro-2-(3-hydroxypropyl)-4-(2-méthylpropyl)amino-2H-thiéno-[3,2-e]-1,2-thiazine-6-sulfonamide 1,1-dioxyde; et (-)-trans-5,6-dihydro-6-(3-méthoxy)propyl-4-propylamino-4H-thiéno[2,3-b]-thiopyranne-2-sulfonamide 7,7-dioxyde.

3. Composition suivant la revendication 2, caractérisée en ce que l'inhibiteur d'anhydrase carbonique est choisi dans le groupe formé par les substances qui suivent :
(R)-3,4-dihydro-4-éthylamino-2-(3-méthoxy)propyl)-2H-thiéno[3,2-e]-1,2-thiazine-6-sulfonamide 1,1-dioxyde et (R)-3,4-dihydro-4-éthylamino-2-(2-méthoxy)éthyl-2H-thiéno[3,2-e]-1,2-thiazine-6-sulfonamide 1,1-dioxyde; (R)-4-éthylamino-3,4-dihydro-2-(4-méthoxy)butyl-2H-thiéno[3,2-e]-1,2-thiazine-6-sulfonamide 1,1-dioxyde; (R)-3,4-dihydro-2-(4-méthoxy)butyl-4-propylamino-2H-thiéno[3,2-e]-1,2-thiazine-6-sulfonamide 1,1-dioxyde; (-)-trans-5,6-dihydro-6-(3-méthoxy)propyl-4-propylamino-4H-thiéno[2,3-b]-thiopyranne-2-sulfonamide 7,7-dioxyde.

4. Composition suivant la revendication 1, caractérisée en ce que l'inhibiteur d'anhydrase carbonique est choisi dans le groupe formé par les substances qui suivent :
(R)-3,4-dihydro-2-(2-éthoxy)éthyl-4-éthylamino-2H-thiéno[3,2-e]-1,2-thiazine-6-sulfonamide 1,1-dioxyde, chlorhydrate; (R)-3,4-dihydro-2-(2-éthoxy)éthyl-4-propylamino-2H-thiéno[3,2-e]-1,2-thiazine-6-sulfonamide 1,1-dioxyde, chlorhydrate; (R)-3,4-dihydro-4-éthylamino-2-(3-méthoxy)propyl-2H-thiéno[3,2-e]-1,2-thiazine-6-sulfonamide 1,1-dioxyde, chlorhydrate; (R)-3,4-dihydro-2-(3-méthoxy)propyl-4-propylamino-2H-thiéno[3,2-e]-1,2-thiazine-6-sulfonamide 1,1-dioxyde, chlorhydrate; (R)-3,4-dihydro-4-éthylamino-2-[2-méthoxyéthoxy)éthyl]-2H-thiéno[3,2-e]-1,2-thiazine-6-sulfonamide 1,1-dioxyde, chlorhydrate; (R)-3,4-dihydro-2-[2-méthoxyéthoxy)éthyl]-4-propylamino-2H-thiéno-[3,2-e]-1,2-thiazine-6-sulfonamide 1,1-dioxyde, chlorhydrate; (R)-3,4-dihydro-4-éthylamino-2-[3-(2-méthoxy)éthoxy)propyl]-2H-thiéno-[3,2-e]-1,2-thiazine-6-sulfonamide 1,1-dioxyde, chlorhydrate; (R)-3,4-dihydro-2-[3-(méthoxyéthoxy)propyl]-4-propylamino-2H-thiéno-[3,2-e]-1,2-thiazine-6-sulfonamide 1,1-dioxyde, chlorhydrate; (R)-3,4-dihydro-4-éthylamino-2-(2-méthoxy)éthyl-2H-thiéno[3,2-e]-1,2-thiazine-6-sulfonamide 1,1-dioxyde, chlorhydrate; (R)-3,4-dihydro-2-(2-méthoxy)éthyl-4-propylamino-2H-thiéno[3,2-e]-1,2-thiazine-6-sulfonamide 1,1-dioxyde, chlorhydrate; (R)-3,4-dihydro-4-éthylamino-2-méthyl-2H-thiéno[3,2-e]-1,2-thiazine-6-sulfonamide 1,1-dioxyde, chlorhydrate; (R)-4-éthylamino-3,4-dihydro-2-(4-méthoxy)butyl-2H-thiéno[3,2-e]-1,2-thiazine-6-sulfonamide 1,1-dioxyde; (R)-3,4-dihydro-2-(4-(méthoxy)butyl-4-propylamino-2-thiéno[3,2-e]-1,2-thiazine-6-sulfonamide 1,1-dioxyde; (R)-4-éthylamino-2-(4-méthoxyphényl)-3,4-dihydro-2H-thiéno[3,2-e]-1,2-thiazine-6-sulfonamide 1,1-dioxyde, chlorhydrate; (R)-4-éthylamino-3,4-dihydro-2-(3-méthoxyphényl)-2H-thiéno[3,2-e]1,2-thiazine-6-sulfonamide 1,1-dioxyde, chlorhydrate; (R)-4-éthylamino-2-(4-hydroxyphényl)-3,4-dihydro-2H-thiéno[3,2-e]-1,2-thiazine-6-sulfonamide 1,1-dioxyde, chlorhydrate; (R)-4-éthylamino-3,4-dihydro-2-(3-hydroxyphényl)-2H-thiéno[3,2-e]-1,2-thiazine-6-sulfonamide 1,1-dioxyde, chlorhydrate; (R)-4-éthylamino-3,4-dihydro-2-(3-hydroxyphénylméthyl)-2H-thiéno-[3,2-e]-1,2-thiazine-6-sulfonamide 1,1-dioxyde, chlorhydrate; (R)-4-éthylamino-3,4-dihydro-2-(3-méthoxyphénylméthyl)-2H-thiéno-[3,2-e]-1,2-thiazine-6-sulfonamide 1,1-dioxyde, chlorhydrate; (R)-4-éthylamino-3,4-dihydro-2-(2-méthylpropyl)-2H-thiéno[3,2-e]-1,2-thiazine-6-sulfonamide 1,1-dioxyde, chlorhydrate; (R)-4-éthylamino-3,4-dihydro-2-(6-hydroxyhexyl)-2H-thiéno[3,2-e]-1,2-thiazine-6-sulfonamide 1,1-dioxyde, chlorhydrate; (R)-3,4-dihydro-2-(3-hydroxypropyl)-4-(2-méthylpropyl)amino-2H-thiéno-[3,2-e]-1,2-thiazine-6-sulfonamide 1,1-dioxyde, chlorhydrate, hémihydrate; et
(-)-trans-5,6-dihydro-6-(3-méthoxy)propyl-4-propylamino-4H-thiéno[2,3-b]-thiopyranne-2-sulfonamide 7,7-dioxyde.

5. Composition suivant la revendication 4, caractérisée en ce que l'inhibiteur d'anhydrase carbonique est choisi dans le groupe formé par les substances qui suivent :
(R)-3,4-dihydro-4-éthylamino-2-(3-méthoxy)propyl-2H-thiéno[3,2-e]-1,2-thiazine-6-sulfonamide 1,1-dioxyde, chlorhydrate, et (R)-3,4-dihydro-4-éthylamino-2-(2-méthoxy)éthyl-2H-thiéno[3,2-e]-1,2-thiazine-6-sulfonamide 1,1-dioxyde, chlorhydrate; (R)-4-éthylamino-3,4-dihydro-2-(4-méthoxy)butyl-2H-thiéno[3,2-e]-1,2-thiazine-6-sulfonamide 1,1-dioxyde; (R)-3,4-dihydro-2-(4-méthoxy)butyl-4-propylamino-2H-thiéno[3,2-e]-1,2-thiazine-6-sulfonamide 1,1-dioxyde; (-)-trans-5,6-dihydro-6-(3-méthoxy)propyl-4-propylamino-4H-thiéno[2,3-b]-thiopyranne-2-sulfonamide 7,7-dioxyde.

6. Composition suivant l'une quelconque des revendications 1 à 5, caractérisée en ce que la concentration en bêta-bloqueur de la composition finale est inférieure ou égale à environ 2,0% en poids et la concentration en inhibiteur d'anhydrase carbonique de la composition finale est inférieure ou égale à environ 5% en poids.

7. Composition suivant la revendication 6, caractérisée en ce que la concentration en bêta-bloqueur de la composition finale varie d'environ 0,1 à environ 1,0% en poids.

8. Composition suivant la revendication 7, caractérisée en ce que la concentration en bêta-bloqueur de la composition finale varie d'environ 0,25 à environ 0,5% en poids.

9. Composition suivant la revendication 8, caractérisée en ce que la concentration en bêta-bloqueur de la composition finale est de 0,25% en poids.

10. Composition suivant la revendication 6, caractérisée en ce que la concentration en inhibiteur d'anhydrase carbonique de la composition finale varie d'environ 0,25 à environ 3% en poids.

11. Composition suivant la revendication 10, caractérisée en ce que la concentration en inhibiteur d'anhydrase carbonique de la composition finale varie d'environ 0,5 à environ 2% en poids.

12. Composition suivant l'une quelconque des revendications 1 à 11, caractérisée en ce que l'on choisit le bêta-bloqueur parmi les formes racémiques et énantiomériques des composés qui suivent : bêtaxolo, timolol, métoprolol, béfunolol, falintolol, lévobunolol, cartéolol, mépindolol, pindolol, bisoprolol, bopindolol, aténolol, arotinolol, acébutolol, nadolol, céliprolol, métipranolol, bévantolol, ICI 118 551, pamatolol, penbutolol, toliprolol, tiprénolol, practolol, porcinolol, exaprolol, cicloprolol, carazolol, tazolol, tiénoxolol, oxprénolol, propranolol, IPS 339, labétolol, dilévalol, esmolol, bupranolol, bunolol, isoxaprolol, diacétolol, hydroxylévobunolol, carvédilol, et leurs sels pharmaceutiquement acceptables.

13. Composition suivant la revendication 12, caractérisée en ce que le bêta-bloqueur est choisi parmi les formes racémiques et énantiomériques des composés qui suivent : bêtaxolol, timolol, cartéolol, lévobunolol et hydroxylévobunolol, et leurs sels pharmaceutiquement acceptables.

14. Composition suivant la revendication 13, caractérisée en ce que le bêta-bloqueur est le bêtaxolol ou un sel pharmaceutiquement acceptable de celui-ci.

15. Composition suivant la revendication 13, caractérisée en ce que le bêta-bloqueur est le S-timolol ou un sel pharmaceutiquement acceptable de celui-ci.

16. Composition suivant l'une quelconque des revendications 1 à 11, caractérisée en ce que le bêta-bloqueur est un thiadiazole de la formule : 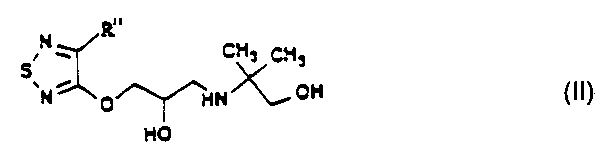 et ses isomères optiquement actifs et sels pharmaceutiquement acceptables de ceux-ci, où R'' est choisi dans le groupe formé par l'hydrogène, les halogènes, les radicaux alkyle en C₁ à C₅, monoalcényle en C₂ à C₅, alcoxy en C₂ à C₅, cycloalkyle en C₁ à C₆, phényle, phénalkyle, morpholino, furyle, thiényle et pyrryle.

17. Composition suivant la revendication 16, caractérisée en ce que R'' est choisi dans le groupe formé par le chlore, les radicaux éthyle, allyle, cyclopropyle, éthoxy, phényle, phénylchlorométhyle et 2-(cyclopropylméthoxy)éthyle.

18. Composition suivant l'une quelconque des revendications 1 à 17, caractérisée en ce qu'elle comprend, en outre, un polymère mucomimétique anionique où la concentration en polymère mucomimétique anionique de la composition finale varie d'environ 0,05 à environ 8,0% en poids.

19. Composition suivant la revendication 18, caractérisée en ce qu'elle comprend, en outre, un substrat véhicule du médicament, finement divisé, où la concentration en substrat véhicule du médicament finement divisé de la composition finale varie d'environ 0,05 à environ 10,0% en poids.

20. Composition suivant l'une quelconque des revendications 1 à 19, à utiliser pour le traitement du glaucome et de l'hypertension oculaire.

21. Utilisation d'un composition comprenant un bêta-bloqueur et un inhibiteur d'anhydrase carbonique dans un véhicule acceptable du point de vue ophtalmique, où la concentration en bêta-bloqueur de la composition finale est inférieure ou égale à environ 2,0% en poids, et la concentration en inhibiteur d'anhydrase carbonique de la composition finale est inférieure ou égale à environ 5% en poids et l'inhibiteur d'anhydrase carbonique répond à la formule : 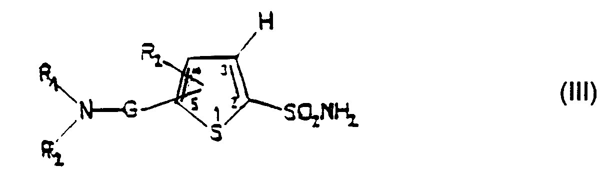 ou un sel pharmaceutiquement acceptable d'un tel composé, structure dans laquelle :
R₁ représente un atome d'hydrogène; un radical alkyle en C₁ à C₄, alkyle en C₂ à C₄ éventuellement substitué par OH, un atome d'halogène, un radical alcoxy en C₁ à C₄ ou C(=O)R₇;
R₂ représente un atome d'hydrogène, un radical alkyle en C₁ à C₈, un radical alkyle en C₃ à C₈ substitué par un OH, un radical NR₅R₆, un atome d'halogène, un radical alcoxy en C₁ à C₄, un radical alcoxy(C₂-C₄)alcoxy(C₁-C₄), un radical OC(=O)R₇ ou C(=O)R₇, un radical alcényle en C₃ à C₇ non substitué ou éventuellement substitué par OH, NR₅R₆, ou alcoxy en C₁ à C₄; un radical alcynyle en C₃ à C₇ non substitué ou éventuellement substitué par OH, NR₅R₆, ou alcoxy en C₁ à C₄; un radical alkyle en C₁ à C₃ substitué par un radical phényle ou R₁₀, chacun d'entre eux pouvant être non substitué ou éventuellement substitué par un radical alkyle en C₁ à C₃, haloalkyle en C₁ à C₃, OH, (CH₂)ₙNR₅R₆, un atome d'halogène, un radical alcoxy en C₁ à C₄, haloalcoxy en C₁ à C₄, C(=O)R₇, S(=O)ₙR₈ ou SO₂NR₅R₆, où m est égal à 0-2 et n est égal à 0-2; un radical alcoxy en C₁ à C₄ éventuellement substitué par NR₅R₆, un atome d'halogène, un radical alcoxy en C₁ à C₄ ou C(=O)R₇, un radical phényle ou R₁₀, chacun d'entre eux pouvant ne pas être substitué ou être substitué éventuellement par un radical alkyle en C₁ à C₃, haloalkyle en C₁ à C₃, OH, (CH₂)ₙNR₅R₆, un atome d'halogène, un radical alcoxy en C₁ à C₄, haloalcoxy en C₁ à C₄, C(=O)R₇, S(=O)ₘR₈ ou SO₂NR₅R₆, où m est 0-2 et n est 0-1, avec la condition que R₁ et R₂ ne représentent pas tous deux des atomes d'hydrogène; ou bien R₁ et R₂ peuvent être réunis pour former un noyau saturé comportant 5 ou 6 atomes choisis parmi O, S, C ou N, tels que, par exemple, un noyau pyrrolidine, oxazolidine, thiomorpholine, thiomorpholine-1,1-dioxyde, morpholine, pipérazine, thiazolidine, 1,1-dioxyde ou tétrahydrooxazine, qui peut ne pas être substitué ou êtresubstitué éventuellement sur du carbone par OH, NR₅R₆, un atome d'halogène, un radical alcoxy en C₁ à C₄, C(=O)R₇, un radical alkyle en C₁ à C₆, un radical alkyle en C₁à C₆ éventuellement substitué par OH, NR₅R₆, un atome d'halogène, un radical alcoxy en C₁ à C₄, C(=O)R₇ ou sur l'azote par NR₅R₆, un radical alcoxy en C₁ à C₄, C(=O)R₇, un radical alkyle en C₁ à C₆ ou alkyle en C₁ à C₆ éventuellement substitué par OH, NR₅R₆, un atome d'halogène, un radical alcoxy en C₁ à C₄ ou C(=O)R₇;
R₃ représente un atome d'hydrogène; un atome d'halogène; un radical alkyle en C₁ à C₄; alcoxy en C₁ à C₈; alkylthiol en C₁ à C₈; alcoxy en C₂ à C₈ éventuellement substitué par OH, NR₅R₆, un atome d'halogène, un radical alcoxy en C₁ à C₄ ou C(=O)R₇; un radical alkyle en C₁ à C₄ éventuellement substitué par R₁; ou bien R₁ et R₃ peuvent être mutuellement liés à des atomes de carbone pour former un cycle à 5 à 7 chaînons dans lequel lesdits atomes de carbone peuvent ne pas être substitués ou être éventuellement substitués par R₄;
R₄ représente un groupe OH; un radical alkyle en C₁ à C₄ non substitué ou éventuellement substitué par OH, NR₅R₆, un atome d'halogène, un radical alcoxy en C₁ à C₄ ou C(=O)R₄; un radical alcoxy en C₁ à C₄; un radical alcoxy en C₂ à C₄ éventuellement substitué par OH, NR₅R₆, un atome d'halogène, un radical alcoxy en C₁ à C₄ ou C(=O)R₇; NR₅R₆; un radical phényle ou R₁₀, chacun d'entre eux n'étant pas substitué ou étant substitué éventuellement par OH, (CH₂)ₙNR₅R₆, un atome d'halogène, un radical alcoxy en C₁ à C₄, haloalcoxy en C₁ à C₄, C(=O)R₇, S(=O)ₘR₆ ou SO₂NR₅R₆, où m est 0-2 et n est 0-2;
avec la condition que lorsque G est SO₂ et que R₃ se trouve en position 4 et représente un atome d'hydrogène ou un atome d'halogène, R₁ et R₂ ne représentent pas d'atomes d'halogène, de radicaux alkyle en C₁ à C₆ éventuellement substitués par OH, de radical alcoxy en C₁ à C₆, de radical alcoxycarbonyle en C₂ à C₆, de radical alcényle en C₂ à C₆, de radical phényle, phénoxy, pyridyle, tétrahydrofuryle, de radical alcanoyle en C₂,à C₆, de radical alcényle en C₂ à C₆, ni qu'ils soient unis pour former un noyau à 5, 6 ou 7 chaînons, saturé ou insaturé, constitué d'atomes choisis parmi C, O, S, N, où ledit azote, lorsqu'il est saturé, est évenuellement substitué par H ou un radical alkyle en C₁ à C₆ ou dans lequel ledit carbone est éventuellement substitué par un radical alkyle en C₁ à C₆, alcoxy en C₁ à C₆ ou OH; et que lorsque R₃ se trouve dans la position 5 et est H, Cl, Br ou alkyle en C₁ à C₃, ni R₁ ni R₂ puissent être de l'ydrogène ou des radicaux alkyle en C₁ à C₄; et que lorsque G est C(=O) et se trouve en position 5 et R₃ représente H, alors ni R₁ ni R₂ ne puissent représenter tous deux CH₃;
R₅ et R₆ sont identiques ou diférents et représentent un atome d'hydrogène; un radical alkyle en C₁ à C₄; alkyle en C₂ à C₄ éventuellement substitué par OH, un atome d'halogène, un radical alcoxy en C₁ à C₄ ou C(=O)R₇; un radical alcoxy en C₁ à C₄; un radical alcoxy en C₂ à C₄ éventuellement substitué par un groupe OH, un atome d'halogène, un radical alcoxy en C₁ à C₄ ou C(=O)R₇; un radical alcényle en C₃ à C₇ non substitué ou éventuellement substitué par OH, NR₅R₆, ou un radical alcoxy en C₁ à C₄; un radical alcynyle en C₃ à C₇ non substitué ou éventuellement substitué par OH, NR₅R₆, ou un radical alcoxy en C₁ à C₄; le radical alkyl(C₁-C₂)cycloalkyle(C₃-C₅); C(=O)R₇ ou bien R₅ et R₆ peuvent être unis pour former un noyau comportant 5 ou 6 atomes choisis parmi O, S, C ou N, comme un noyau pyrrolidine, oxazolidine, thiomorpholine, thiomorpholine-1,1-dioxyde, morpholine, pipérazine ou thiazolidine-1,1-dioxyde, qui peut ne pas être substitué ou être éventuellement substitué sur un atome de carbone par un groupe OH, (=O), un atome d'halogène, un radical alcoxy en C₁ à C₄, C(=O)R₇, un radical alkyle en C₁ à C₆, un radical alkyle C₁ à C₆ éventuellement substitué par un groupe OH, un atome d'halogène, un radical alcoxy en C₁ à C₄, C(=O)R₇, ou sur l'atome d'azote par un radical alcoxy en C₁ à C₄, C(=O)R₇, S(=O)ₙR₉, un radical alkyle en C₁ à C₆ ou un radical alkyle en C₂ à C₆ éventuellement substitué par un groupe OH, una tome d'halogène, un radical alcoxy en C₁ à C₄, C(=O)R₇, ou sur l'atome de soufre par un groupe (=O)ₘ, où m est égal à 0-2;
R₇ représente un groupe alkyle en C₁ à C₈; un radical alkyle en C₁ à C₆ éventuellement substitué par un groupe OH, NR₅R₆, un atome d'halogène, un radical alcoxy en C₁ à C₄ ou C(=O)R₉; un radical alcoxy en C₁ à C₄; un radical alcoxy en C₂ à C₄ éventuellement substitué par un groupe OH, NR₅R₆, un atome d'halogène ou un radical alcoxy en C₁ à C₄, NR₅R₆, ou un radical phényle ou R₁₀, chacun d'entre eux pouvant ne pas être substitué ou être substitué éventuellement par un groupe OH, un atome d'halogène, un radicla alkyle en C₁ à C₃, haloalcoxy en C₁ à C₃, (CH₂)ₙNR₅R₆, S(=O)ₘR₉ ou SO₂NR₅R₆, où n est égal à 0 ou à 1 et m est égal à 0-2;
R₈ représente un groupe alkyle en C₁ à C₄; un groupe alkyle éventuellement substitué par OH, NR₅R₆, un atome d'halogène, un radical alcoxy en C₁ à C₄ ou C(=O)R₇;
R₉ représente un radical alkyle en C₁ à C₄, un radical alcoxy en C₁ à C₄; un radical amino, un radical alkylamino en C₁ à C₃, ou un radical dialkylamino en C₁ à C₃;
R₁₀ représente un système à noyau monocyclique de 5 ou 6 atomes composés de C, N, O et/ou S,comme le furanne, le thiophène, le pyrrole, le pyrazole, l'imidazole, le triazole, le tétrazole, l'oxazole, l'isoxazole, l'isothiazole, le thiazole, le thiadiazole, la pyridine, la pyrimidine, la pyridazine et la pyrazine; et
G représente C(=O) ou SO₂, ou bien
où l'inhibiteur d'anhydrase carbonique est le (-)-trans-5,6-dihydro-6-(3-méthoxy)propyl-4-propylamino-4H-thiéno[2,3-b]-thiopyranne-2-sulfonamide 7,7-dioxyde, en vue de la préparation d'un médicament destiné au traitement du glaucome et de l'hypertension oculaire.

22. Utilisation suivant la revendication 21, caractérisée en ce que l'inhibiteur d'anhydrase carbonique est choisi dans le groupe formé par les substances qui suivent :
(R)-3,4-dihydro-2-(2-éthoxy)éthyl-4-éthylamino-2H-thiéno[3,2-e]-1,2-thiazine-6-sulfonamide 1,1-dioxyde; (R)-3,4-dihydro-2-(2-éthoxy)éthyl-4-propylamino-2H-thiéno[3,2-e]-1,2-thiazine-6-sulfonamide 1,1-dioxyde; (R)-3,4-dihydro-4-éthylamino-2-(3-méthoxy)propyl-2H-thiéno[3,2-e]-1,2-thiazine-6-sulfonamide 1,1-dioxyde; (R)-3,4-dihydro-2-(3-méthoxy)propyl-4-propylamino-2H-thiéno[3,2-e]-1,2-thiazine-6-sulfonamide 1,1-dioxyde; (R)-3,4-dihydro-4-éthylamino-2-[2-méthoxyéthoxy)éthyl]-2H-thiéno[3,2-e]-1,2-thiazine-6-sulfonamide 1,1-dioxyde; (R)-3,4-dihydro-2-[2-méthoxyéthoxy)éthyl]-4-propylamino-2H-thiéno-[3,2-e]-1,2-thiazine-6-sulfonamide 1,1-dioxyde; (R)-3,4-dihydro-4-éthylamino-2-[3-(2-méthoxyéthoxy)propyl]-2H-thiéno-[3,2-e]-1,2-thiazine-6-sulfonamide 1,1-dioxyde; (R)-3,4-dihydro-2-[3-(méthoxyéthoxy)propyl]-4-propylamino-2H-thiéno-[3,2-e]-1,2-thiazine-6-sulfonamide 1,1-dioxyde; (R)-3,4-dihydro-4-éthylamino-2-(2-méthoxy)éthyl-2H-thiéno[3,2-e]-1,2-thiazine-6-sulfonamide 1,1-dioxyde; (R)-3,4-dihydro-2-(2-méthoxy)éthyl-4-propylamino-2H-thiéno[3,2-e]-1,2-thiazine-6-sulfonamide 1,1-dioxyde; (R)-3,4-dihydro-4-éthylamino-2-méthyl-2H-thiéno[3,2-e]-1,2-thiazine-6-sulfonamide 1,1-dioxyde; (R)-4-éthylamino-3,4-dihydro-2-(4-méthoxy)butyl-2H-thiéno[3,2-e]-1,2-thiazine-6-sulfonamide 1,1-dioxyde; (R)-3,4-dihydro-2-(4-(méthoxy)butyl-4-propylamino-2-thiéno(3,2-e]-1,2-thiazine-6-sulfonamide 1,1-dioxyde; (R)-4-éthylamino-2-(4-méthoxyphényl)-3,4-dihydro-2H-thiéno[3,2-e]-1,2-thiazine-6-sulfonamide 1,1-dioxyde; (R)-4-éthylamino-3,4-dihydro-2-(3-méthoxyphényl)-2H-thiéno(3,2-e]-1,2-thiazine-6-sulfonamide 1,1-dioxyde; (R)-4-éthylamino-2-(4-hydroxyphényl)-3,4-dihydro-2H-thiéno[3,2-e]-1,2-thiazine-6-sulfonamide 1,1-dioxyde; (R)-4-éthylamino-3,4-dihydro-2-(3-hydroxyphényl)-2H-thiéno[3,2-e]-1,2-thiazine-6-sulfonamide 1,1-dioxyde; (R)-4-éthylamino-3,4-dihydro-2-(3-hydroxyphénylméthyl)-2H-thiéno-[3,2-e]-1,2-thiazine-6-sulfonamide 1,1-dioxyde; (R)-4-éthylamino-3,4-dihydro-2-(3-méthoxyphénylméthyl)-2H-thiéno-[3,2-e]-1,2-thiazine-6-sulfonamide 1,1-dioxyde; (R)-4-éthylamino-3,4-dihydro-2-(2-méthylpropyl)-2H-thiéno[3,2-e]-1,2-thiazine-6-sulfonamide 1,1-dioxyde; (R)-4-éthylamino-3,4-dihydro-2-(6-hydroxyhexyl)-2H-thiéno[3,2-e]-1,2-thiazine-6-sulfonamide 1,1-dioxyde; (R)-3,4-dihydro-2-(3-hydroxypropyl)-4-(2-méthylpropyl)amino-2H-thiéno-[3,2-e]-1,2-thiazine-6-sulfonamide 1,1-dioxyde; et (-)-trans-5,6-dihydro-6-(3-méthoxy)propyl-4-propylamino-4H-thiéno[2,3-b]-thiopyranne-2-sulfonamide 7,7-dioxyde.

23. Utilisation suivant la revendication 21, caractérisée en ce que l'inhibiteur d'anhydrase carbonique est choisi dans le groupe formé par les substances qui suivent :
(R)-3,4-dihydro-4-éthylamino-2-(3-méthoxy)propyl)-2H-thiéno(3,2-e]-1,2-thiazine-6-sulfonamide 1,1-dioxyde, et (R)-3,4-dihydro-4-éthylamino-2-(2-méthoxy)éthyl-2H-thiéno[3,2-e]-1,2-thiazine-6-sulfonamide 1,1-dioxyde; (R)-4-éthylamino-3,4-dihydro-2-(4-méthoxy)butyl-2H-thiéno[3,2-e]-1,2-thiazine-6-sulfonamide 1,1-dioxyde; (R)-3,4-dihydro-2-(4-méthoxy)butyl-4-propylamino-2H-thiéno[3,2-e]-1,2-thiazine-6-sulfonamide 1,1-dioxyde; (-)-trans-5,6-dihydro-6-(3-méthoxy)propyl-4-propylamino-4H-thiéno[2,3-b]-thiopyranne-2-sulfonamide 7,7-dioxyde.

24. Utilisation suivant la revendication 21, caractérisée en ce que l'inhibiteur d'anhydrase carbonique est choisi dans le groupe formé par les substances qui suivent :
(R)-3,4-dihydro-2-(2-éthoxy)éthyl-4-éthylamino-2H-thiéno[3,2-e]-1,2-thiazine-6-sulfonamide 1,1-dioxyde, chlorhydrate; (R)-3,4-dihydro-2-(2-éthoxy)éthyl-4-propylamino-2H-thiéno[3,2-e]-1,2-thiazine-6-sulfonamide 1,1-dioxyde, chlorhydrate; (R)-3,4-dihydro-4-éthylamino-2-(3-méthoxy)propyl-2H-thiéno[3,2-e]-1,2-thiazine-6-sulfonamide 1,1-dioxyde, chlorhydrate; (R)-3,4-dihydro-2-(3-méthoxy)propyl-4-propylamino-2H-thiéno[3,2-e]1,2-thiazine-6-sulfonamide 1,1-dioxyde, chlorhydrate; (R)-3,4-dihydro-4-éthylamino-2-[2-méthoxyéthoxy)éthyl]-2H-thiéno[3,2-e]-1,2-thiazine-6-sulfonamide 1,1-dioxyde, chlorhydrate; (R)-3,4-dihydro-2-[2-méthoxyéthoxy)éthyl]-4-propylamino-2H-thiéno-[3,2-e]-1,2-thiazine-6-sulfonamide 1,1-dioxyde, chlorhydrate; (R)-3,4-dihydro-4-éthylamino-2-[3-(2-méthoxy)éthoxy)propyl]-2H-thiéno-[3,2-e]-1,2-thiazine-6-sulfonamide 1,1-dioxyde, chlorhydrate; (R)-3,4-dihydro-2-[3-(méthoxyéthoxy)propyl]-4-propylamino-2H-thiéno-[3,2-e]-1,2-thiazine-6-sulfonamide 1,1-dioxyde, chlorhydrate; (R)-3,4-dihydro-4-éthylamino-2-(2-méthoxy)éthyl-2H-thiéno[3,2-e]-1,2-thiazine-6-sulfonamide 1,1-dioxyde, chlorhydrate; (R)-3,4-dihydro-2-(2-méthoxy)éthyl-4-propylamino-2H-thiéno[3,2-e]-1,2-thiazine-6-sulfonamide 1,1-dioxyde, chlorhydrate; (R)-3,4-dihydro-4-éthylamino-2-méthyl-2H-thiéno[3,2-e]-1,2-thiazine-6-sulfonamide 1,1-dioxyde, chlorhydrate; (R)-4-éthylamino-3,4-dihydro-2-(4-méthoxy)butyl-2H-thiéno[3,2-e]-1,2-thiazine-6-sulfonamide 1,1-dioxyde; (R)-3,4-dihydro-2-(4-méthoxy)butyl-4-propylamino-2-thiéno[3,2-e]-1,2-thiazine-6-sulfonamide 1,1-dioxyde; (R)-4-éthylamino-2-(4-méthoxyphényl)-3,4-dihydro-2H-thiéno[3,2-e]-1,2-thiazine-6-sulfonamide 1,1-dioxyde, chlorhydrate; (R)-4-éthylamino-3,4-dihydro-2-(3-méthoxyphényl)-2H-thiéno[3,2-e]-1,2-thiazine-6-sulfonamide 1,1-dioxyde, chlorhydrate; (R)-4-éthylamino-2-(4-hydroxyphényl)-3,4-dihydro-2H-thiéno[3,2-e]-1,2-thiazine-6-sulfonamide 1,1-dioxyde, chlorhydrate; (R)-4-éthylamino-3,4-dihydro-2-(3-hydroxyphényl)-2H-thiéno[3,2-e]-1,2-thiazine-6-sulfonamide 1,1-dioxyde, chlorhydrate; (R)-4-éthylamino-3,4-dihydro-2-(3-hydroxyphénylméthyl)-2H-thiéno-[3,2-e]-1,2-thiazine-6-sulfonamide 1,1-dioxyde, chlorhydrate; (R)-4-éthylamino-3,4-dihydro-2-(3-méthoxyphénylméthyl)-2H-thiéno-[3,2-e]-1,2-thiazine-6-sulfonamide 1,1-dioxyde, chlorhydrate; (R)-4-éthylamino-3,4-dihydro-2-(2-méthylpropyl)-2H-thiéno[3,2-e]-1,2-thiazine-6-sulfonamide 1,1-dioxyde, chlorhydrate; (R)-4-éthylamino-3,4-dihydro-2-(6-hydroxyhexyl)-2H-thiéno[3,2-e]-1,2-thiazine-6-sulfonamide 1,1-dioxyde, chlorhydrate; (R)-3,4-dihydro-2-(3-hydroxypropyl)-4-(2-méthylpropyl)amino-2H-thiéno-[3,2-e]-1,2-thiazine-6-sulfonamide 1,1-dioxyde, chlorhydrate, hémihydrate; et
(-)-trans-5,6-dihydro-6-(3-méthoxy)propyl-4-propylamino-4H-thiéno[2,3-b]-thiopyranne-2-sulfonamide 7,7-dioxyde.

25. Utilisation suivant la revendication 21, caractérisée en ce que l'inhibiteur d'anhydrase carbonique est choisi dans le groupe formé par les substances qui suivent :
(R)-3,4-dihydro-4-éthylamino-2-(3-méthoxy)propyl-2H-thiéno[3,2-e]-1,2-thiazine-6-sulfonamide 1,1 dioxyde, chlorhydrate, et (R)-3,4-dihydro-4-éthylamino-2-(2-méthoxy)éthyl-2H-thiéno[3,2-e]-1,2-thiazine-6-sulfonamide 1,1-dioxyde, chlorhydrate; (R)-4-éthylamino-3,4-dihydro-2-(4-méthoxy)butyl-2H-thiéno[3,2-e]-1,2-thiazine-6-sulfonamide 1,1-dioxyde; (R)-3,4-dihydro-2-(4-méthoxy)butyl-4-propylamino-2H-thiéno[3,2-e]-1,2-thiazine-6-sulfonamide 1,1-dioxyde; (-)-trans-5,6-dihydro-6-(3-méthoxy)propyl-4-propylamino-4H-thiéno[2,3-b]-thiopyranne-2-sulfonamide 7,7-dioxyde.

26. Utilisation suivant l'une quelconque des revendications 21 à 25, caractérisée en ce que l'on choisit le bêta-bloqueur parmi les formes racémiques et énantiomériques des composés qui suivent :
bêtaxolol, timolol, métoprolol, béfunolol, falintolol, lévobunolol, cartéolol, mépindolol, pindolol, bisoprolol, bopindolol, aténolol, arotinolol, acébutolol, nadolol, céliprolol, métipranolol, bévantolol, ICI 118 551, pamatolol, penbutolol, toliprolol, tiprénolol, practolol, porcinolol, exaprolol, cicloprolol, carazolol, tazolol, tiénoxolol, oxprénolol, propranolol, IPS 339, labétolol, dilévalol, esmolol, bupranolol, bunolol, isoxaprolol, diacétolol, hydroxylévobunolol, et leurs sels pharmaceutiquement acceptables.

27. Utilisation suivant la revendication 26, caractérisée en ce que l'on choisit le bêta-bloqueur dans le groupe formé par les substances qui suivent : bêtaxolol, timolol, cartéolol, lévobunolol et hydroxylévobunolol, et leurs sels pharmaceutiquement acceptables.

28. Utilisation suivant l'une quelconque des revendications 21 à 27, caractérisée en ce que le bêta-bloqueur est un thiadizaole de la formule : 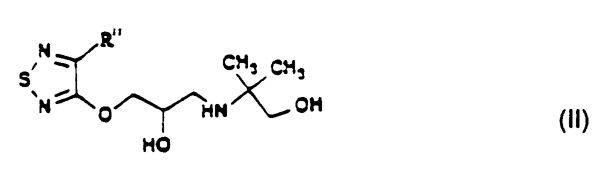 et ses isomères optiquement actifs et sels pharmaceutiquement acceptables de ceux-ci, où R'' est choisi dans le groupe formé par l'hydrogène, les halogènes, les radicaux alkyle en C₁ à C₅, monoalcényle en C₂ à C₅, alcoxy en C₂ à C₅, cycloalkyle en C₃ à C₆, phényle, phénalkyle, morpholino, furyle, thiényle et pyrryle.

29. Utilisation suivant la revendication 28, caractérisée en ce que R'' est choisi dans le groupe formé par le chlore, les radicaux éthyle, allyle, cyclopropyle, éthoxy, phényle, phénylchlorométhyle et 2-(cyclopropylméthoxy)éthyle.
